(19) Europäisches Patentamt
European Patent Office
Office européen des brevets

(11) EP 0 955 301 A2

(12) EUROPEAN PATENT APPLICATION

(43) Date of publication:
**10.11.1999 Bulletin 1999/45**

(51) Int Cl.6: **C07D 487/08**, A61K 31/44
// (C07D487/08, 209:00, 209:00)

(21) Application number: **99302306.8**

(22) Date of filing: **25.03.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **27.04.1998 US 83108 P**

(71) Applicant: **Pfizer Products Inc.**
**Groton, Connecticut 06340 (US)**

(72) Inventors:
- **Yohannes, Daniel**
  **Groton, Connecticut 06340 (US)**
- **Bundesmann, Mark Werner**
  **Mystic, Connecticut 06355 (US)**

(74) Representative:
**Simpson, Alison Elizabeth Fraser et al**
**Urquhart-Dykes & Lord,**
**91 Wimpole Street**
**London W1M 8AH (GB)**

(54) **7-aza-bicyclo[2.2.1]-heptane derivatives, their preparation and use according to their affinity for neuronal nicotinic acetylchloline receptors**

(57) Compounds of the formula

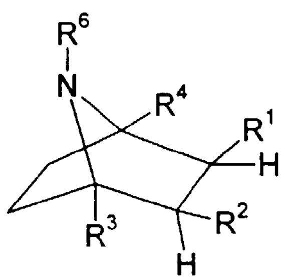

I

and their pharmaceutically acceptable salts, wherein $R^1$, $R^2$, $R^3$ and $R^4$ are defined as in the specification, intermediates in the synthesis of such compounds, pharmaceutical compositions containing such compounds and methods of using such compounds in the treatment of neurological and psychological disorders are claimed.

EP 0 955 301 A2

## Description

Background of the Invention

This invention relates to 7-hetero-bicyclo[2.2.1]-heptanes, as defined more specifically by formula I below. Compounds of formula I bind to neuronal nicotinic acetylcholine specific receptor sites and are useful in modulating cholinergic function. Such compounds are useful in the treatment of inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amylotropic lateral sclerosis (ALS), cognitive dysfunction, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke, traumatic brain injury (TBI), obsessive-compulsive disorder, psychosis, Huntington's Chorea, tardive dyskinesia, hyperkinesia, dyslexia, schizophrenia, multi-infarct dementia, age related cognitive decline, epilepsy, including petit mal absence epilepsy, senile dementia of the Alzheimer's type (AD), Parkinson's disease (PD), attention deficit hyperactivity disorder (ADHD) and Tourette's Syndrome.

The compounds of this invention may also be used in combination with an antidepressant such as a tricyclic antidepressant or a serotonin reuptake inhibiting antidepressant (SRI), in order to treat both the cognitive decline and depression associated with AD, PD, stroke, Huntington's Chorea or traumatic brain injury (TBI); in combination with muscarinic agonists in order to stimulate both central muscarinic and nicotinic receptors for the treatment, for example, of ALS, cognitive dysfunction, age related cognitive decline, AD, PD, stroke, Huntington's Chorea and TBI; in combination with neurotrophic factors such as NGF in order to maximize cholinergic enhancement for the treatment, for example, of ALS, cognitive dysfunction, age related cognitive decline, AD, PD stroke, Huntington's Chorea and TBI: or in combination with agents that slow or arrest AD such as cognition enhancers, amyloid aggregation inhibitors, secretase inhibitors, tau kinase inhibitors, neuronal antiinflammatory agents and estrogen-like therapy.

Other compounds that bind to neuronal nicotinic receptor sites are referred to in United States Patent Application 08/963,852, which was filed on November 4, 1997.

In Devop of the Future, 1997, 22 (11): 1210-1220, Donglu Bai et al., reviews methods of synthesizing epibatidine and the pharmacological properties of epibatidine.

Epibatidine derivatives and their various pharmacological activities are referred to, inter alia, in the following references: United States patent application 845,042, filed March 3, 1992; Japanese patent application JP 6312989A2, published November 8, 1994; World patent application WO 95/03306, published February 2, 1995; Japanese patent application JP 7010878A2, published January 13, 1995; Japanese patent application 7033771A2, published February 3, 1995. World patent application 95/07078A1, published March 16, 1995; United States patent US 5,346,906, issued September 13, 1994; European patent application EP 657455A1, published June 14, 1994; Japanese patent application JP 7061940A2, published March 7, 1995; European patent application EP 664293A1, published July 26, 1995; World patent application WO 94/22868A1, published October 13, 1994; and World patent application WO 96/06093, published February 29, 1996.

Summary of the Invention

This invention relates to aryl fused azapolycyclic compounds of the formula

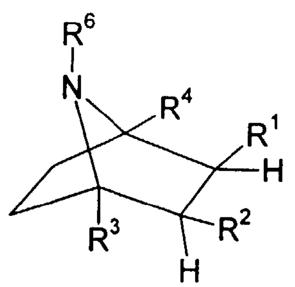

I

wherein

$R^1$, $R^2$, $R^3$ and $R^4$ are selected, independently from hydrogen, $-CO_2R^5$, aryl and heteroaryl, wherein said aryl is

selected from phenyl and naphthyl and said heteroaryl is selected from pyrazinyl, benzofuranyl, quinolyl, isoquinolyl, benzothienyl, isobenzofuryl, pyrazolyl, indolyl, isoindolyl, benzimidazolyl, purinyl, carbazolyl, 1,2,5-thiadiazolyl, quinazolinyl, pyridazinyl, pyrazinyl, cinnolinyl, phthalazinyl, quinoxalinyl, xanthinyl, hypoxanthinyl, pteridinyl, 5-azacytidinyl, 5-azauracilyl, triazolopyridinyl, imidazolopyridinyl, pyrrolopyrimidinyl, and pyrazolopyrimidinyl oxazolyl, isoxazoyl, thiazolyl, isothiazolyl, furanyl, pyrazolyl, pyrrolyl, tetrazolyl, triazolyl, thienyl, imidazolyl, pyridinyl, and pyrimidinyl, and wherein said phenyl and said heteroaryl may optionally be substituted with from one to three substitutuents, and are preferably substituted with one or two substutituents, independently selected form ($C_1$-$C_6$) alkyl optionally substituted with from one to seven (preferably with from zero to four) fluorine atoms, halo (i.e., chloro, fluoro, bromo or iodo), phenyl, benzyl, hydroxy, acetyl, amino, cyano, nitro, ($C_1$-$C_6$)alkoxy optionally substituted with from one to seven (preferably with from zero to four) fluorine atoms, ($C_1$-$C_6$)alkylamino and [($C_1$-$C_6$) alkyl]$_2$amino;

$R^5$ is ($C_1$-$C_6$) alkyl, aryl, heteroaryl, ($C_1$-$C_4$)alkylene-aryl and ($C_1$-$C_4$)alkylene-heteroaryl, wherein said aryl and heteroaryl are defined as above, and wherein said ($C_1$-$C_6$)alkyl may optionally be substituted with from one to three substituents independently selected from halo, ($C_1$-$C_6$)alkyl, ($C_1$-$C_6$ )alkoxy, ($C_1$-$C_4$)alkoxy-($C_1$-$C_4$)alkyl, amino, ($C_1$-$C_6$)alkylamino, and [($C_1$-$C_6$)alkyl]$_2$amino; and

$R^6$ is hydrogen or ($C_1$-$C_6$)alkyl;

with the proviso that: (a) at least one of $R^1$, $R^2$, $R^3$, and $R^4$ must be aryl or heteraryl; (b) when neither $R^1$ nor $R^2$ is hydrogen, $R^1$ and $R^2$ are in the "exo" configuration; (c) $R^1$ and $R^2$ can not both be -$CO_2R^5$; (d) if either $R^3$ or $R^4$ is -$CO_2R^5$ and $R^5$ is an alkyl or alkoxyalkyl group, then one of $R^1$ and $R^2$ must be aryl or heteroaryl; and (e) if either $R^1$ or $R^2$ is -$CO_2R^5$ and $R^5$ is an alkyl or alkoxyalkyl group, then one of $R^3$ and $R^4$ must be aryl or heteroaryl;

and the pharmaceutically acceptable salts of such compounds.

Preferred compounds of this invention include compounds of the formula I, and their pharmaceutically acceptable salts, wherein one of $R^1$ and $R^2$ is optionally substituted phenyl and the other is hydrogen, and wherein $R^3$ and $R^4$ are hydrogen.

More preferred compounds of this invention are compounds of the formula I, and their pharmaceutically acceptable salts, wherein one of $R^1$ and $R^2$ is phenyl substituted with fluoro or nitro and the other is hydrogen, and wherein $R^3$ and $R^4$ are hydrogen.

More specific preferred embodiments of this invention are compounds of the formula I, and their pharmaceutically acceptable salts, wherein $R^3$ and $R^4$ are hydrogen and one $R^1$ and $R^2$ is hydrogen and the other is: (a) 3-fluorophenyl; (b) 4-nitrophenyl; or 3-fluoro-4-nitrophenyl.

Other embodiments of this invention relate to the following compounds of the formula I and their pharmaceutically acceptable salts:

2β-(3,4-difluorophenyl)-7-aza-bicyclo[2.2.1]heptane;
2β-(3,5-dichlorobenzene)-7-aza-bicyclo[2.2.1]heptane;
2β-(4-nitrophenyl)-7-aza-bicyclo[2.2.1]heptane;
2β-(3-thiophene)-7-aza-bicyclo[2.2.1]heptane;
2β-(3-fluoro-4-chlorophenyl)-7-aza-bicyclo[2.2.1]heptane;
2β-(3-flourophenyl)-7-aza-bicyclo[2.2.1]heptane;
2β-(3-hydroxyphenyl)-7-aza-bicyclo[2.2.1]heptane;
2β-(3-acetophenone)-7-aza-bicyclo[2.2. 1]heptane;
2β-(4-trifluoromethylphenyl)-7-aza-bicyclo[2.2.1]heptane;
2β-(3-fluoro-4-methylphenyl)-7-aza-bicyclo[2.2.1]heptane;
2β-(3-chlorophenyl)-7-aza-bicyclo[2.2.1]heptane;
2β-(n-benzyl-5-pyridonyl)-7-aza-bicyclo[2.2.1]heptane;
2β-(n-methyl-5-pyridonyl)- 7-aza-bicyclo[2.2.1]heptane;
2β-(3-fluoro-5-nitrophenyl)-7-aza-bicyclo[2.2.1]heptane;
2β-(4-aminophenyl)-7-aza-bicyclo[2.2.1]heptane;
2β-(3-fluoro-4-trifluoromethyl-phenyl)-7-aza-bicyclo[2.2.1]heptane;
2β-(4-chlorophenyl)-7-aza-bicyclo[2.2.1]heptane;
2β-(3,4-methylenedioxyphenyl)-7-aza-bicyclo[2.2.1]heptane;
2β-(2-chloro-6-methyl-5-pyridinyl)-7-aza-bicyclo[2.2.1]heptane;
2β-(4-cyanophenyl)-7-aza-bicyclo[2.2.1]heptane;
2β-(3-fluoro-4-nitro-phenyl)-7-aza-bicyclo[2.2.1]heptane;
2β-(4-amido-phenyl)-7-aza-bicyclo[2.2.1]heptane;
2β-(3-fluoro-4-amino-phenyl)-7-aza-bicyclo[2.2.1]heptane;
2β-(4-sulfonamido-phenyl)-7-aza-bicyclo[2.2.1]heptane;

2β-(3-methyl-5-isoxzazole)-7-aza-bicyclo[2.2.1]heptane;
2β-(3-methyl-5-isoxzazole)-7-aza-bicyclo[2.2.1]heptane, N-methyl;
2β-(3-methyl-5-isoxzazole)-7-aza-bicyclo[2.2.1]heptane, N-acetyl;
2b-(3,4-difluorophenyl)-7-azabicyclo[2.2.1]heptane;
4-(7-aza-bicyclo[2.2.1]hept-2-yl)-benzamidine;
2-(4-methanesulfonyl-phenyl)-7-aza-bicyclo[2.2.1]heptane;
4-(7-aza-bicyclo[2.2.1]hept-2-yl)-phenol;
2-(4-methylsulfanyl-phenyl)-7-aza-bicyclo[2.2.1]heptane;
4-(7-aza-bicyclo[2.2.1]hept-2-yl)-benzoic acid methyl ester;
4-(7-aza-bicyclo[2.2.1]hept-2-yl)-benzoic acid;
2-(3-fluoro-4-tetrazol-1-yl-phenyl)-7-aza-bicyclo[2.2.1]heptane;
2-(4-nitro-3-trifluoromethyl-phenyl)-7-aza-bicyclo[2.2.1]heptane;
2-[3-fluoro-4-(5-trifluoromethyl-tetrazol-1-yl)-phenyl]-7-aza-bicyclo[2.2.1]heptane;
2-(3-chloro-4-nitro-phenyl)-7-aza-bicyclo[2.2.1]heptane;
2-(4-tetrazol-1-yl-phenyl)-7-aza-bicyclo[2.2.1]heptane;
2-(6-methoxy-pyridin-2-yl)-7-aza-bicyclo[2.2.1]heptane;
2-(4-methanesulfinyl-phenyl)-7-aza-bicyclo[2.2.1]heptane;
2-(4-bromo-3-fluoro-phenyl)-7-aza-bicyclo[2.2.1]heptane;
2-(4-cyano-3-fluoro-phenyl)-7-aza-bicyclo[2.2.1]heptane;
2-(3,4,5-trifluoro-phenyl)-7-aza-bicyclo[2.2.1]heptane;
2-(3,4,5-trimethoxy-phenyl)-7-aza-bicyclo[2.2.1]heptane;
2-(5-nitro-furan-2-yl)-7-aza-bicyclo[2.2.1]heptane;
5-(7-aza-bicyclo[2.2.1]hept-2-yl)-3-methyl-benzo[d]isoxazole;
6-(7-aza-bicyclo[2.2.1]hept-2-yl)-3-methyl-benzo[d]isoxazole;
6-(7-aza-bicyclo[2.2.1]hept-2-yl)-1,4-dihydro-quinoxaline-2,3-dione;
6-(7-aza-bicyclo[2.2.1]hept-2-yl)-quinoxaline; and
1-[4-(7-aza-bicyclo[2.2.1]hept-2-yl)-2-fluoro-phenyl]-ethanone.

Examples of specific compounds of the formula I are the following:

7-Azabicyclo[2.2.1]heptane, 2-(5-methyl-3-isoxazolyl)-;
7-Azabicyclo[2.2.1]heptane, 2-[5-(trifluoromethyl)-3-isoxazolyl]-;
7-Azabicyclo[2.2.1]heptane, 2-(5-chloro-3-isoxazolyl)-;
7-Azabicyclo[2.2.1]heptane, 2-(5-methyl-3-isothiazolyl)-;
7-Azabicyclo[2.2.1]heptane, 2-[5-(trifluoromethyl)-3-isothiazolyl]-;
7-Azabicyclo[2.2.1]heptane, 2-(5-chloro-3-isothiazolyl)-;
7-Azabicyclo[2.2.1]heptane, 2-(2-fluoro-1*H*-imidazol-4-yl)-;
7-Azabicyclo[2.2.1]heptane, 2-[2-(trifluoromethyl)-1*H*-imidazol-4-yl]-;
7-Azabicyclo[2.2.1]heptane, 2-(2-chloro-1*H*-imidazol-4-yl)-;
7-Azabicyclo[2.2.1]heptane, 2-(2-methyl-1*H*-imidazol-4-yl)-;
7-Azabicyclo[2.2.1]heptane, 2-[5-(trifluoromethyl)-1*H*-tetrazol-1-yl]-;
7-Azabicyclo[2.2.1]heptane, 2-(5-fluoro-1*H*-tetrazol-1-yl)-;
7-Azabicyclo[2.2.1]heptane, 2-(5-chloro-1*H*-tetrazol-1-yl)-;
7-Azabicyclo[2.2.1]heptane, 2-[5-(trifluoromethyl)-1*H*-1,2,4-triazol-3-yl]-;
7-Azabicyclo[2.2.1]heptane, 2-(5-fluoro-1*H*-1,2,4-triazol-3-yl)-;
7-Azabicyclo[2.2.1]heptane, 2-(5-chloro-1*H*-1,2,4-triazol-3-yl)-;
7-Azabicyclo[2.2.1]heptane, 2-(5-methyl-1*H*-1,2,4-triazol-3-yl)-;
7-Azabicyclo[2.2.1]heptane, 2-(1*H*-tetrazol-5-yl)-;
7-Azabicyclo[2.2.1]heptane, 2-(1*H*-1,2,3-triazol-4-yl)-;
7-Azabicyclo[2.2.1]heptane, 2-(1*H*-pyrrol-2-yl)-;
7-Azabicyclo[2.2.1]heptane, 2-[5-(trifluoromethyl)-1,3,4-thiadiazol-2-yl]-;
7-Azabicyclo[2.2.1]heptane, 2-(5-fluoro-1,3,4-thiadiazol-2-yl)-;
7-Azabicyclo[2.2.1]heptane, 2-(5-chloro-1,3,4-thiadiazol-2-yl)-;
7-Azabicyclo[2.2.1]heptane, 2-(5-methyl-1,3,4-thiadiazol-2-yl)-;
7-Azabicyclo[2.2.1]heptane, 2-[5-(trifluoromethyl)-1,3,4-oxadiazol-2-yl]-;
7-Azabicyclo[2.2.1]heptane, 2-(5-fluoro-1,3,4-oxadiazol-2-yl)-;
7-Azabicyclo[2.2.1]heptane, 2-(5-chloro-1,3,4-oxadiazol-2-yl)-;
7-Azabicyclo[2.2.1]heptane, 2-(5-methyl-1,3,4-oxadiazol-2-yl)-;

7-Azabicyclo[2.2.1]heptane, 2-(5-methyl-1*H*-pyrazol-3-yl)-;
7-Azabicyclo[2.2.1]heptane, 2-[5-(trifluoromethyl)-1*H*-pyrazol-3-yl]-;
7-Azabicyclo[2.2.1]heptane, 2-(5-chloro-1*H*-pyrazol-3-yl)-;
7-Azabicyclo[2.2.1]heptane, 2-(2-methyl-5-oxazolyl)-;
7-Azabicyclo[2.2.1]heptane, 2-[2-(trifluoromethyl)-5-oxazolyl]-;
7-Azabicyclo[2.2.1]heptane, 2-(2-chloro-5-oxazolyl)-;
7-Azabicyclo[2.2.1]heptane, 2-(2-fluoro-5-oxazolyl)-;
7-Azabicyclo[2.2.1]heptane, 2-(2-methyl-5-thiazolyl)-;
7-Azabicyclo[2.2.1]heptane, 2-[2-(trifluoromethyl)-5-thiazolyl]-;
7-Azabicyclo[2.2.1]heptane, 2-(2-chloro-5-thiazolyl)-;
7-Azabicyclo[2.2.1]heptane, 2-(2-fluoro-5-thiazolyl)-;
Ethanone, 1-[4-(7-azabicyclo[2.2.1]hept-2-yl)-2-fluorophenyl]-2,2,2-trifluoro-;
7-Azabicyclo[2.2.1]heptane, 2-[2-(4-pyridinyl)ethenyl]-, (*E*)-;
7-Azabicyclo[2.2.1]heptane, 2-[2-(3-pyridinyl)ethenyl]-, (*E*)-;
7-Azabicyclo[2.2.1]heptane, 2-[2-(5-pyrimidinyl)ethenyl]-, (*E*)-;
7-Azabicyclo[2.2.1]heptane, 2-[2-(4-pyridazinyl)ethenyl]-, (*E*)-;
2(3*H*)-Benzoxazolone, 6-(7-azabicyclo[2.2.1]hept-2-yl)-4-fluoro-;
2(3*H*)-Benzothiazolone, 6-(7-azabicyclo[2.2.1]hept-2-yl)-4-fluoro-;
2*H*-Indol-2-one, 5-(7-azabicyclo[2.2.1]hept-2-yl)-7-fluoro-1,3-dihydro-;
2*H*-Benzimidazol-2-one,6-(7-azabicyclo[2.2.]hept-2-yl)-4-fluoro-1,3-dihydro-;
2*H*-Benzimidazol-2-one,6-(7-azabicyclo[2.2.1]hept-2-yl)-4-fluoro-1,3-dihydro-1-methyl-;
Ethanone, 1-[4-(7-azabicyclo[2.2.1]hept-2-yl)-2-fluorophenyl]-;
7-Azabicyclo[2.2.1]heptane, 2-(3-pyridinylethynyl)-;
7-Azabicyclo[2.2.1]heptane, 2-(4-pyridinylethynyl)-;
7-Azabicyclo[2.2.1]heptane, 2-(4-pyridazinylethynyl)-;
7-Azabicyclo[2.2.1]heptane, 2-(5-pyrimidinylethynyl)-;
2(3*H*)-Benzoxazolone, 6-(7-azabicyclo[2.2.1]hept-2-yl)-;
2(3*H*)-Benzothiazolone, 6-(7-azabicyclo[2.2.1]hept-2-yl)-;
2*H*-Indol-2-one, 5-(7-azabicyclo[2.2.1]hept-2-yl)-1,3-dihydro-;
2*H*-Benzimidazol-2-one, 5-(7-azabicyclo[2.2.1]hept-2-yl)-1,3-dihydro-;
2*H*-Benzimidazol-2-one, 6-(7-azabicyclo[2.2.1]hept-2-yl)-1,3-dihydro-1-methyl-;
1-Propanone, 1-[4-(7-azabicyclo[2.2.1]hept-2-yl)-2-fluorophenyl]-3,3,3-trifluoro-;
7-Azabicyclo[2.2.1]heptane, 2-(4-azido-3-fluorophenyl)-;
Phenol, 5-(7-azabicyclo[2.2.1]hept-2-yl)-2-nitro-;
7-Azabicyclo[2.2.1]heptane, 2-(4-nitrocyclohexyl)-;
7-Azabicyclo[2.2.1]heptane, 2-(4-nitrobicyclo[2.2.2]oct-1-yl)-:
7-Azabicyclo[2.2.1]heptane, 2-[(6-chloro-3-pyridinyl)ethynyl]-:
7-Azabicyclo[2.2.1]heptane, 2-[2-(6-chloro-3-pyridinyl)ethenyl]-, (*E*)-;
1,5-Methano-8*H*-pyrido[1,2-a][1,5]diazocin-8-one, 9-(7-azabicyclo[2.2.1]hept-2-yl)-1,2,3,4,5,6-hexahydro-;
2(1*H*)-Pyridinone, 3-(7-azabicyclo[2.2.1]hept-2-yl)-1-methyl-: and
2(1*H*)-Pyridinone, 3-(7-azabicyclo[2.2.1]hept-2-yl)-.

This invention also relates to the pharmaceutically acceptable acid addition salts of the compounds of formula I. Examples of pharmaceutically acceptable acid addition salts of the compounds of formula I are the salts of hydrochloric acid, p-toluenesulfonic acid, fumaric acid, citric acid, succinic acid, salicylic acid, oxalic acid, hydrobromic acid, phosphoric acid, methanesulfonic acid, tartaric acid, malate, di-p-toluoyl tartaric acid, and mandelic acid.

Unless otherwise indicated, the term "halo", as used herein, includes fluoro, chloro, bromo and iodo.

Unless otherwise indicated, the term "alkyl", as used herein, may be straight, branched or cyclic, and may include straight and cyclic moieties as well as branched and cyclic moieties.

Unless otherwise indicated, the term "one or more substituents", as used herein, refers to from one to the maximum number of substituents possible based on the number of available bonding sites.

The term "treatment", as used herein, refers to reversing, alleviating, inhibiting the progress of, or preventing the disorder or condition to which such term applies, or one or more symptoms of such condition or disorder. The term "treatment", as used herein, refers to the act of treating, as "treating" is defined immediately above.

The compounds of formula I may have optical centers and therefore may occur in different enantiomeric configurations. This invention includes all enantiomers, diastereomers, and other stereoisomers of such compounds of formula I, as well as racemic and other mixtures thereof.

The present invention also relates to all radiolabelled forms of the compounds of the formulae I. Preferred

radiolabelled compounds of formula I are those wherein the radiolabels are selected from as $^3H$, $^{11}C$, $^{14}C$, $^{18}F$, $^{123}I$ and $^{125}I$. Such radiolabelled compounds are useful as research and diagnostic tools in metabolism pharmacokinetics studies and in binding assays in both animals and man.

The present invention also relates to a pharmaceutical composition for use in reducing nicotine addiction or aiding in the cessation or lessening of tobacco use in a mammal, including a human, comprising an amount of a compound of the formula I, or a pharmaceutically acceptable salt thereof, that is effective in reducing nicotine addiction or aiding in the cessation or lessening of tobacco use and a pharmaceutically acceptable carrier.

The present invention also relates to a method for reducing nicotine addiction or aiding in the cessation or lessening of tobacco use in a mammal, including a human, comprising administering to said mammal an amount of a compound of the formula I, or a pharmaceutically acceptable salt thereof, that is effective in reducing nicotine addiction or aiding in the cessation or lessening of tobacco use.

The present invention also relates to a method of treating a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amylotropic lateral sclerosis (ALS), cognitive dysfunction, hypertension, bulimia, anorexia, obesity, cardiac anythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke, traumatic brain injury (TBI), psychosis, Huntington's Chorea, tardive dyskinesia, hyperkinesia, dyslexia, schizophrenia, multi-infarct dementia, age related cognitive decline, epilepsy, including petit mal absence epilepsy, senile dementia of the Alzheimer's type (AD), Parkinson's disease (PD), attention deficit hyperactivity disorder (ADHD) and Tourette's Syndrome in a mammal, comprising administering to a mammal in need of such treatment an amount of a compound of the formula I, or a pharmaceutically acceptable salt thereof, that is effective in treating such disorder or condition.

The present invention also relates to a pharmaceutical composition for treating a disorder or condition selected from inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amylotropic lateral sclerosis (ALS), cognitive dysfunction, hypertension, bulimia, anorexia, obesity, cardiac arrythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbituates, opioids or cocaine), headache, stroke, traumatic brain injury (TBI), psychosis, Huntington's Chorea, tardive dyskinesia, hyperkinesia, dyslexia, schizophrenia, multi-infarct dementia, age related cognitive decline, epilepsy, including petit mal absence epilepsy, senile dementia of the Alzheimer's type (AD), Parkinson's disease (PD), attention deficit hyperactivity disorder (ADHD) and Tourette's Syndrome in a mammal, comprising an amount of a compound of the formula I, or a pharmaceutically accepable salt thereof, and a pharmaceutically acceptable carrier.

This invention also relates to a process for preparing a compound of the formula

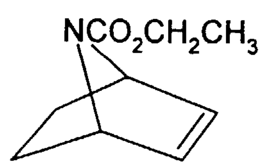

XVI

comprising reacting a compound of the formula

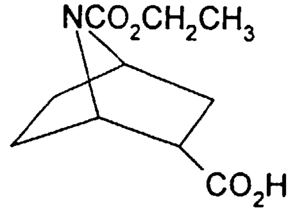

XVII

with lead tetraacetate and copper acetate. This reaction is preferably conducted in a reaction inert solvent such as benzene, toluene or xylenes, at a temperature from about room temperature to about the reflux temperature of the

solvent, preferably at about the reflux temperature.

Detailed Description of the Invention

Except where otherwise stated, $R^1$ through $R^6$ and structural formulas I, IX and X in the reaction schemes and discussion that follow are defined as above.

SCHEME 1

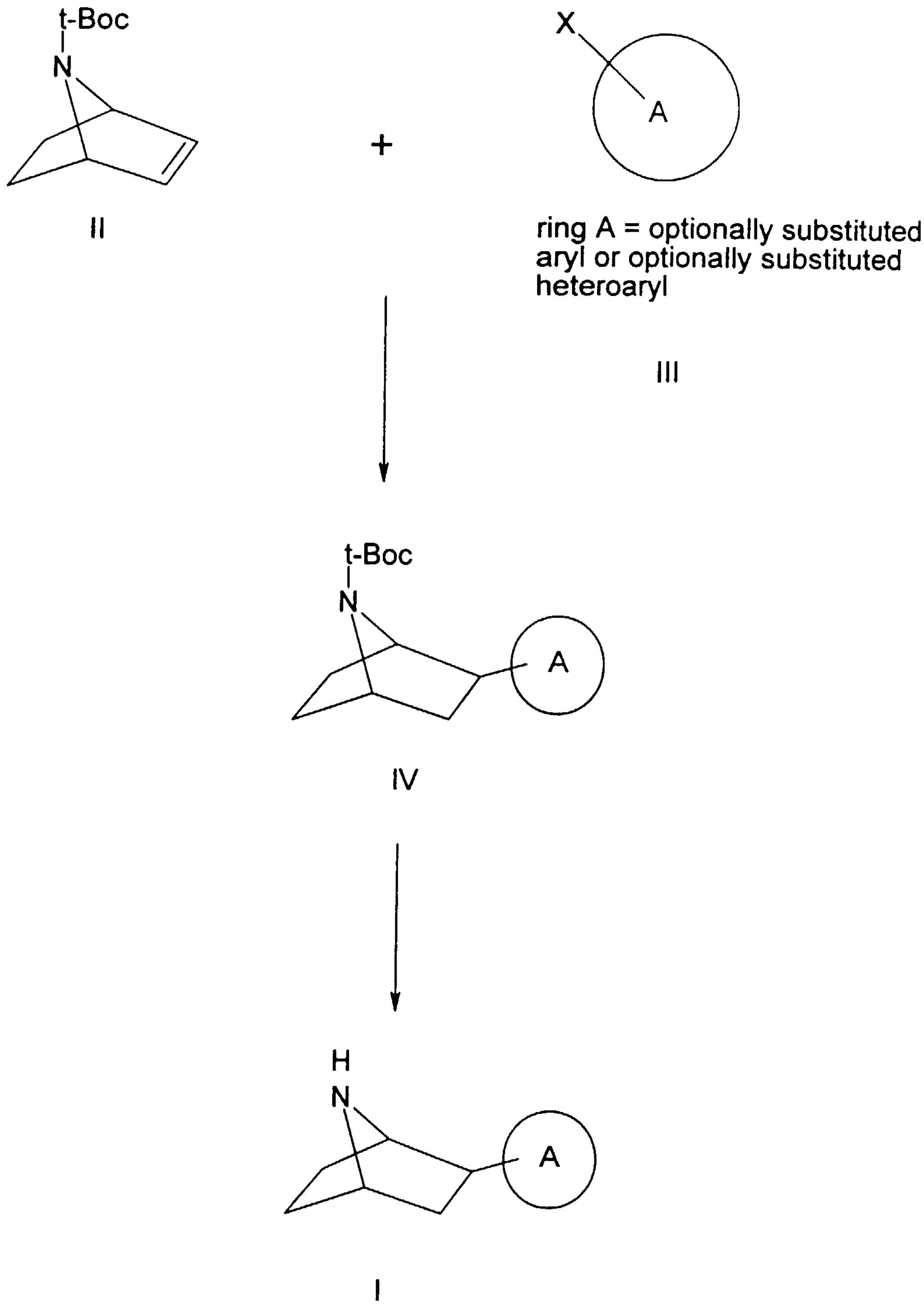


II

III

IV

I

SCHEME 2

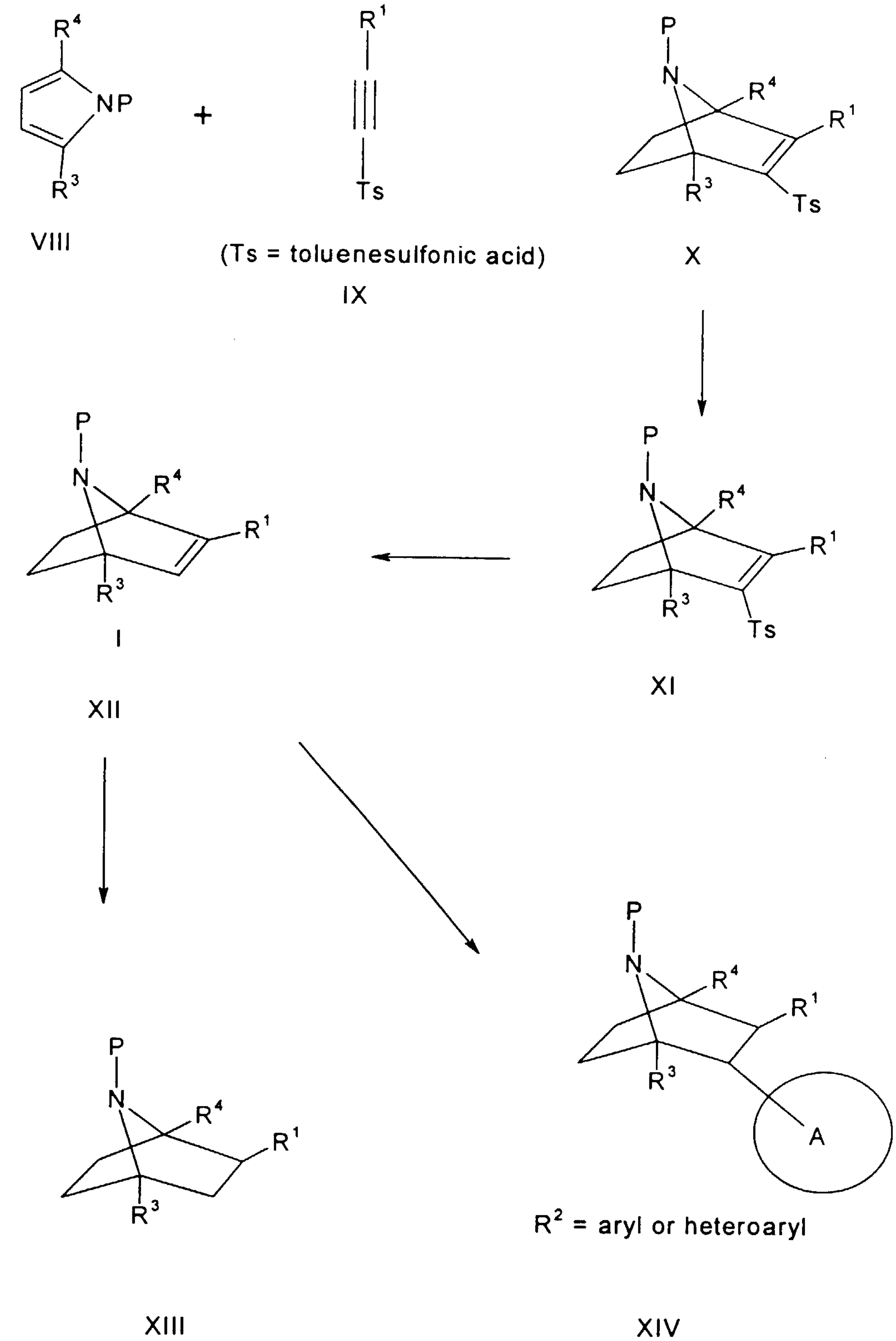

VIII

(Ts = toluenesulfonic acid)

IX

X

I

XII

XI

XIII

$R^2$ = aryl or heteroaryl

XIV

SCHEME 3

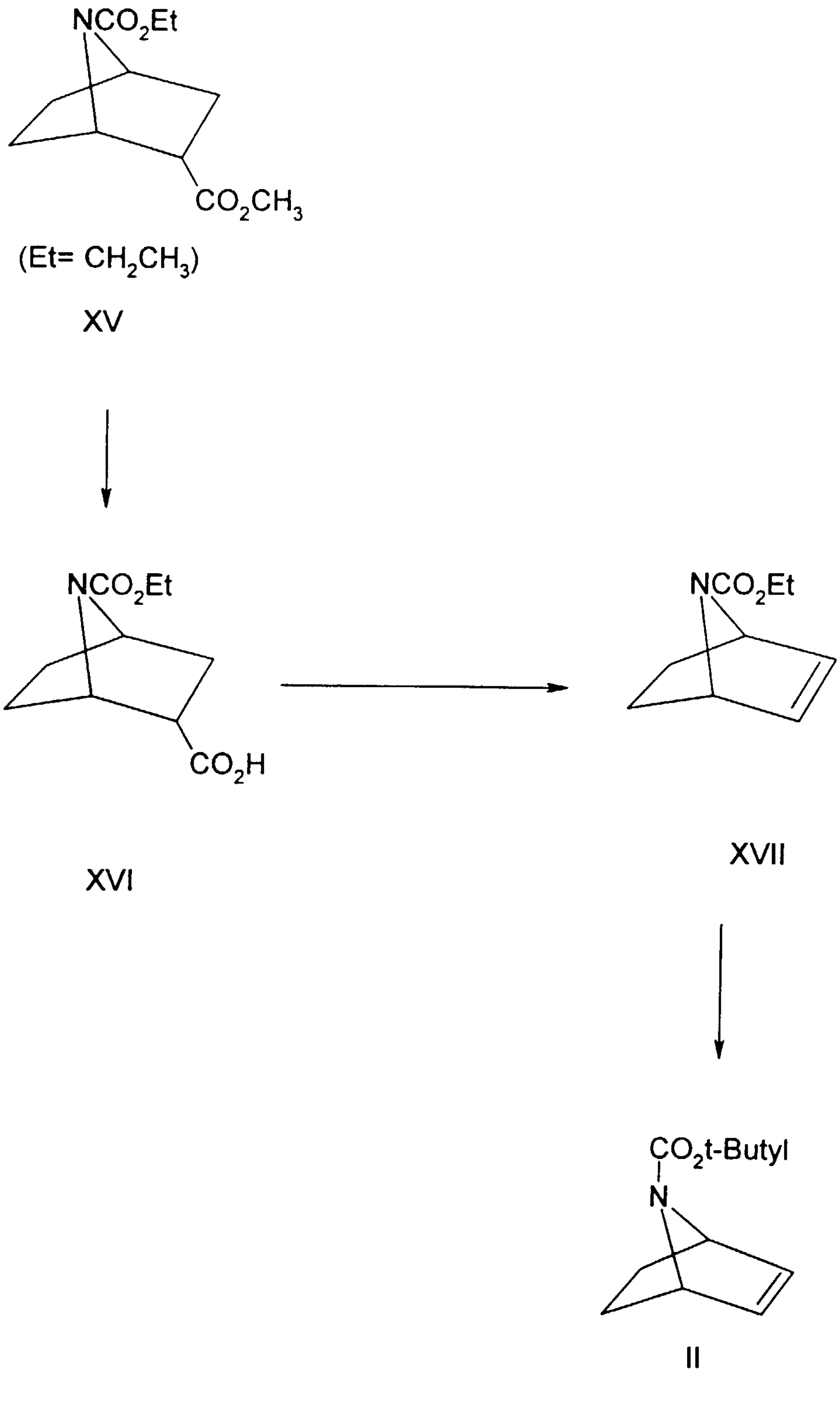

(Et= $CH_2CH_3$)

XV

XVI

XVII

II

Scheme 1 illustrates the preparation of compounds of the formula I wherein $R^2$ is an optionally substituted phenyl or heteroaryl group and all of $R^1$, $R^3$ and $R^4$ are hydrogen. Referring to Scheme 1, the compound of formula II, prepared as illustrated in Scheme 2 and described below, or prepared as described by Altenbach, H. J. et al., Chim. Berichte, 1991, 124, 791-801, is reacted with a compound of the formula III, wherein X is bromine or iodine and ring A is an optionally substituted aryl or heteroaryl group, to form the nitrogen protected compound of formula IV. This reaction, which is a reductive Heck coupling, is typically conducted in a reaction inert polar solvent such as N,N-dimethylformamide (DMF), THF or acetonitrile, preferably DMF, in the presence of formic acid, a secondary amine base such as piperidine, and a catalytic amount of palladium tetrakistriphenylphosphine or another suitable palladium (O) catalyst. The reaction temperature can range from about 25°C to about 120°C, preferably at the lowest possible temperature at which the aryl or heteroaryl halide will react with the palladium catalyst in a reasonable amount of time. For most reactions, room temperature for 24-72 hours up to about 4-5 days provide the desired reaction conditions, although higher temperatures may be used to increase the rate of reaction.

Removal of the nitrogen protecting group from the compound of formula IV using standard methods that are well known to those of skilled in the art yields the desired compound of formula I. For example, reaction of the compound of formula IV with hydrochloric acid in ethyl acetate gives the unprotected hydrochloric salt of the corresponding compound of the formula I, and reaction of the compound of formula IV with trfluoroacetic acid in methylene chloride yields the unprotected trifluroacetic acid salt of the same.

Protecting groups other than t-Boc, which is shown in Schemes 1 and 2, can also be used. Appropriate alternative nitrogen protecting groups (e.g., include $-COCF_3$, $-COCCl_3$, $-COOCH_2CCl_3$, $-COO(C_1-C_6)$alkyl and $-COOCH_2C_6H_5$ and methods of adding and removing them will be obvious to those skill in the art. (See T. W. Green and G. M. Wets, "Protective Group in Organic Synthesis", "1991, John Wiley & Sons, New York, N.Y.).

The process of Scheme 1 is described in greater detail in United States Patent 5,565,573, which is incorporated herein by reference in its entirety.

Scheme 2 illustrates a method of preparing all compounds of the formula I, including those which can be prepared using the procedure of Scheme 1. Referring to Scheme 2, a compound of the formula VIII, wherein P is a nitrogen protecting group, is reacted with a compound of the formula IX, wherein Ts is toluenesulfonic acid, to form the corresponding compound of formula X. Alternatively, benzenesulfonic acid may be used instead of toluenesulfonic acid in this reaction. Suitable nitrogen protecting groups will be obvious to those skill in the art (see T. W. Greene and G. M. Wots, "Protective Groups in Organic Synthesis", 1991, John Wiley & Sons, New York, N.Y.) and include $(C_1-C_6)$ alkyl groups and groups having the formula -COR wherein R is $-N(C_1-C_6)$alkyl, $(C_1-C_6)$alkyl or $-O-(C_1-C_6)$alkyl. This reaction is typically conducted neat at a temperature of about 80°C to about 85°C.

The compound of formula X that is produced in the foregoing reaction is then converted into the corresponding compound of formula XI by hydrogenating it in an acetonitrile solvent at a temperature from about 15°C to about 90°C, preferably at about room temperature, using methods well known to those of skill in the art (e.g., under a hydrogen gas pressure of about 1-3 atmospheres and using a palladium on carbon (Pd/C) catalyst or other palladium catalyst). Removal of the toluenesulfonic acid or benzenesulfonic acid group from the compound of formula XI yields the corresponding compound of formula XII. This can be accomplished by reacting the compound of formula XI with a sodium mercury amalgam (6%) in methanol and tetrahydrofuran (THF), in the presence of sodium hydrophosphate ($Na_2HPO_4$) and sodium dihydrophosphate ($NaH_2PO_4$). Preferably, the reactants are mixed at a temperature of about-78°C and then allowed to warm to room temperature.

The compound of the formula XII can be converted into the corresponding compound having formula XIII by subjecting it to a hydrogenation reaction as described above. The compound of formula XII can then be converted into the corresponding compound having formula XIV, wherein $R^2$ is an aryl or heteraryl group, using the methods described above and illustrated in Scheme 1.

Removal of the nitrogen protecting group from compounds of the formula XIII and XIV, as described above, yields the corresponding final products of formula I.

Scheme 3 illustrates a method of preparing the t-Boc protected olefin that is the starting material used in the process of Scheme 1. Referring to Scheme 3, the starting material of formula VIII can be obtained as described by D. Bai. et al., J. Org. Chem., 1996, 61: 4600-6. This ester is then hydrolyzed, using methods well known to those of skill in the art, to form the corresponding carboxylic acid of formula IX.

Reaction of the compound of formula IX with lead tetraacetate and copper acetate yields the compound of formula X. This reaction is generally conducted in a reaction inert solvent such as benzene, toluene, or zylenes, at a temperature from about room temperature to about the reflux temperature of the solvent. It is preferably conducted in benzene at the reflux temperature in an inert atmosphere (e.g., a nitrogen or argon atmosphere).

The desired nitrogen protected intermediate of formula II can be then be obtained by reacting the compound of formula X with tetramethylsilyl iodide (TMSI) and trifluoroacetic acid, in the presence of triethylamine (TEA), followed by reaction with t-butylpyrocarbonate, also in the presence of TEA. Both these reactions are typically conducted in a reaction inert solvent such as chloroform, methylene chloride, dichloroethane or another chlorinated hydrocarbon solvent, preferably chloroform, at a temperature from about room temperature to about the reflux temperature of the solvent, preferably at the reflux temperature.

Compounds of the formula I wherein $R^6$ is $(C_1-C_6)$ alkyl can be prepared from the corresponding compounds wherein $R^6$ is hydrogen using standard alkylation and reductive amination methods well known to those of skill in the art. (See Jung et al., J.C.S. Chem. Commun., 1984, 10, 630-32; Fletcher et al., J. Org. Chem., 1994 59, 1971-78; Mariano et al., J. A. C. S., 1981, 103, 3148-60, and Gonzales et al., J. A. C. S., 1995, 117, 3405-21).

Syntheses of olefins identical to that of formula II except that the nitrogen is protected by a protecting group other than t-Boc are described by Altenbach et al., Angew Chem. Suppl., 1982,1614-1221, and by Clayton et al., Tetrahedron Letters, 1993, 34, 7493.

The compounds of the formula I which are basic in nature are capable of forming a wide variety of different salts with various inorganic and organic acids. Although such salts must be pharmaceutically acceptable for administration to animals, it is often desirable in practice to initially isolate a compound of the formula I from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent such as methanol or ethanol. Upon careful evaporation of the solvent, the desired solid salt is obtained.

The acids which are used to prepare the pharmaceutically acceptable acid addition salts of the base compounds of this invention are those which form non-toxic acid addition salts, i.e., salts containing pharmacologically acceptable anions, such as hydrochloride, hydrobromide, hydroiodide, nitrate, sulfate or bisulfate, phosphate or acid phosphate, acetate, lactate, citrate or acid citrate, tartrate or bitartrate, succinate, maleate, fumarate, gluconate, saccharate, benzoate, methanesulfonate and pamoate [i.e., 1,1'-methylene-bis-(2-hydroxy-3-naphthoate)] salts.

In each of the reactions discussed above, or illustrated in Schemes 1-3, above, pressure is not critical unless otherwise indicated. Pressures from about 0.5 atmospheres to about 5 atmospheres are generally acceptable, with ambient pressure, i.e., about 1 atmosphere, being preferred as a matter of convenience.

The compounds of the formula I and their pharmaceutically acceptable salts (hereafter the active compounds") can be administered via either the oral, transdermal (e.g., through the use of a patch), intranasal, sublingual, rectal, parenteral or topical routes. Transdermal and oral administration are preferred. These compounds are, most desirably, administered in dosages ranging from about 0.25 mg up to about 1500 mg per day, preferably from about 0.25 to about 300 mg per day in single or divided doses, although variations will necessarily occur depending upon the weight and condition of the subject being treated and the particular route of administration chosen. However, a dosage level that is in the range of about 0.01 mg to about 10 mg per kg of body weight per day is most desirably employed. Variations may nevertheless occur depending upon the weight and condition of the persons being treated and their individual responses to said medicament, as well as on the type of pharmaceutical formulation chosen and the time period and interval during which such administration is camed out. In some instances, dosage levels below the lower limit of the aforesaid range may be more than adequate, while in other cases still larger doses may be employed without causing any harmful side effects, provided that such larger doses are first divided into several small doses for administration throughout the day.

The active compounds can be administered alone or in combination with pharmaceutically acceptable carriers or diluents by any of the several routes previously indicated. More particularly, the active compounds can be administered in a wide variety of different dosage forms, e.g., they may be combined with various pharmaceutically acceptable inert carriers in the form of tablets, capsules, transdermal patches, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents. In addition, oral pharmaceutical compositions can be suitably sweetened and/or flavored. In general, the active compounds are present in such dosage forms at concentration levels ranging from about 5.0% to about 70% by weight

For oral administration, tablets containing various excipients such as microcrystalline cellulose, sodium citrate, calcium carbonate, dicalcium phosphate and glycine may be employed along with various disintegrants such as starch (preferably com, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc can be used for tabletting purposes. Solid compositions of a similar type may also be employed as fillers in gelatin capsules; preferred materials in this connection also include lactose or milk sugar] as well as high molecular weight polyethylene glycols. When aqueous suspensions and/or elixirs are desired for oral administration the active ingredient may be combined with various sweetening or flavoring agents, coloring matter and, if so desired, emulsifying and/or suspending agents, together with such diluents as water, ethanol, propylene glycol, glycerin and various combinations thereof.

For parenteral administration, a solution of an active compound in either sesame or peanut oil or in aqueous propylene glycol can be employed. The aqueous solutions should be suitably buffered (preferably pH greater than 8), if necessary, and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

It is also possible to administer the active compounds topically and this can be done by way of creams, a patch, jellies, gels, pastes, ointments and the like, in accordance with standard pharmaceutical practice.

Biological Assay

The effectiveness of the active compounds in suppressing nicotine binding to specific receptor sites is determined by the following procedure which is a modification of the methods of Lippiello, P. M. and Femandes, K. G. (in The Binding of L-[$^3$H]Nicotine To A Single Class of High-Affinity Sites in Rat Brain Membranes, Molecular Pharm., 29, 448-54, (1986)) and Anderson, D. J. and Americ, S. P. (in Nicotinic Receptor Binding of $^3$H-Cystisine $^3$H-Nicotine and $^3$H-Methylcarmbamylcholine In Rat Brain, European J. Pharm., 253, 261-67 (1994)).

Procedure

Male Sprague-Dawley rats (200-300 g) from Charles River were housed in groups in hanging stainless steel wire cages and were maintained on a 12 hour light/dark cycle (7 a.m.-7 p.m. light period). They received standard Purina Rat Chow and water *ad libitum.*

The rats were killed by decapitation. Brains were removed immediately following decapitation. Membranes were prepared from brain tissue according to the methods of Lippiello and Femandez (Molec Pharmacol, 29, 448-454, (1986) with some modifications. Whole brains were removed, rinsed with ice-cold buffer, and homogenized at 0° in 10 volumes of buffer (w/v) using a Brinkmann Polytron™, setting 6, for 30 seconds. The buffer consisted of 50 mM Tns HCl at a pH of 7.5 at room temperature. The homogenate was sedimented by centrifugation (10 minutes; 50,000 x g; 0 to 4°C. The supematant was poured off and the membranes were gently resuspended with the Polytron and centrifuged again (10 minutes; 50,000 x g; 0 to 4°C. After the second centrifugation, the membranes were resuspended in assay buffer at a concentration of 1.0g/100mL. The composition of the standard assay buffer was 50 mM Tris HCl, 120 mM NaCl, 5 mM KCl, 2 mM $MgCl_2$, 2 mM $CaCl_2$ and has a pH of 7.4 at room temperature.

Routine assays were performed in borosilicate glass test tubes. The assay mixture typically consisted of 0.9 mg of membrane protein in a final incubation volume of 1.0 mL. Three sets of tubes were prepared wherein the tubes in each set contained 50µL of vehicle, blank, or test compound solution, respectively. To each tube was added 200 µL of [$^3$H]-nicotine in assay buffer followed by 750 µL of the membrane suspension. The final concentration of nicotine in each tube was 0.9 nM. The final concentration of cytisine in the blank was 1 µM. The vehicle consisted of deionized water containing 30 µL of 1 N acetic acid per 50 mL of water. The test compounds and cytisine were dissolved in vehicle. Assays were initiated by vortexing after addition of the membrane suspension to the tube. The samples were incubated at 0 to 4° C in an iced shaking water bath. Incubations were terminated by rapid filtration under vacuum through Whatman GF/B™ glass fiber filters using a Brandel™ multi-manifold tissue harvester. Following the initial filtration of the assay mixture, filters were washed two times with ice-cold assay buffer (5 m each). The filters were then placed in counting vials and mixed vigorously with 20 ml of Ready Safe™ (Beckman) before quantification of radioactivity. Samples were counted in a LKB Wallach Rackbeta™ liquid scintillation counter at 40-50% efficiency. All determinations were in triplicate.

Calculations

Specific binding (C) to the membrane is the difference between total binding in the samples containing vehicle only and membrane (A) and non-specific binding in the samples containing the membrane and cytisine (B), i.e.,

$$\text{Specific binding} = (C) = (A) - (B).$$

Specific binding in the presence of the test compound (E) is the difference between the total binding in the presence of the test compound (D) and non-specific binding (B), i.e., (E) = (D) - (B).

$$\% \text{ Inhibition} = (1-((E)/(C)) \text{ times } 100.$$

The compounds of the invention that were tested in the above assay exhibited $IC_{50}$ values of less than 1 µM.

The following experimental examples illustrate, but do not limit the scope of, this invention.

In the Examples, below, the melting points are not corrected. NMR spectra were recorded on a Varian spectrometer at 400 MHz unless otherwise noted. Spectra chemical shifts are reported in δ relative to chloroform ($CHCl_3$), methanol ($CH_3OH$), or dimethylsulfoxide (DMSO). IR spectra were obtained as a potassium bromide press. HRMS

was performed by M-Scan Inc. in a matrix of *m*-nitro benzyl alcohol and PEG 200 or 300 using a cesium ion gun.

## EXAMPLE 1

2β-(3,4-DIFLUOROPHENYL)-7-AZA-BICYCLO[2.2.1]HEPTANE, HYDROCHLORIC ACID SALT

A. To a stirred solution of N-t-BOC- azanorbornene (0.4 mmol., 1.0 equivalent (equiv.)) in N,N-dimethylformamide (DMF) (0.4M) under nitrogen gas ($N_2$) at room temperature (RT), was added piperidine (1.4 mmol., 3.5 equiv.), followed by formic acid (1 mmol., 2.5 equiv.) and 3,4 difluoroiodobenzene (0.6 mmol., 1.5 equiv.). The reaction mixture was stirred until homogeneous and then palladium diacetate di(triphenylphosphine) ($Pd(OAc)_2(Ph_3P)_2$) (0.02 mmol., 0.05 equiv.) was added. The reaction mixture was then purged with $N_2$ and heated to 80-90°C for fifteen hours whereby a black precipitate formed. The reaction mixture was then partitioned between 100 ml ethyl acetate and 30 ml water ($H_2O$). The organic layer was then separated and washed, once with 20 ml sodium bicarbonate, twice with 40 ml water and once with 30 ml brine. The organic layer was dried over sodium sulfate ($Na_2SO_4$), filtered, and the solvents were removed *in vacuo* to yield N-t-BOC-2b-(3,4-difluorophenyl)-7-aza-bicyclo [2.2.1]heptane an oil, which was purified by flash chromatograohy (200 mesh silica, 20g, 96/4 hexanes/ethyl acetate) (42mg/50% yield).
B. The t-BOC protecting group was removed by treatment of the above product with 4 ml of 2.5 M HCl in ethyl acetate at RT for 2.5 hours. Removal of the solvent and excess HCl *in vacuo* results in an oil that is titriated with ethyl acetate to yield white crystals of the title product. (22.5mg/67% yield). MP 206.5-208.5°C.

IR: 2992.7, 2953.8, 2929.1, 2882.0, 2827.2, 2717.0, 2653.3, 2547.4, 1434.1, 1373.1, 1358.9, 1281.1, 1121.1, 888.2, 823.1, 763.4 $cm^{-1}$.
MS: CI (m/z) 210 ($M+H^+$).
HRMS (m/z) 210.1102, calculated for $C_{12}H_{14}NF_2$, 210.1094.
$^1H$ NMR ($CDCl_3$) δ 9.91(1H, br s), 9.32 (1H, br s), 7.32-7.12 (3H, m), 4.39 (1H, s), 4.08 (1H. d, J=3.5 Hz), 3.1 (1H, dd, J=8.8, 6.7 Hz), 2.35-2.17 (4H, m), 1.81 (1H, ap. t, J=7.1, 11.6Hz), 1.7 (1H, ap. t, J=11.8, 8.5 Hz).
$^{13}C$ NMR ($CDCl_3$) δ 137.5, 123.4, 117.8, 117.6, 117.1, 116.8, 63.8, 58.5, 45.7, 37.1, 28.7, 25.5 .

The compounds of Examples 2-27 were prepared according the method of Example 1 using the appropriate reactants. The title compounds of Examples 2-51 were prepared using speed analoging technology, as described below. High speed analoging was accomplished in a 96 well plate that used six wells for standards. An automated robot dispensed solutions to a vial in each well. To each vial was added 50 ml of a 0.1M solution of a unique aryl iodide (1.0 equiv.) in N,N-dimethylformamide (DMF). Then 25 ml of a 0.3M solution of azanorbornene in DMF was added, followed by 9 ml a solution that consisted of ammonium formate (1.38M, 2.5 equiv.) and potasium acetate (1.94M, 3.5 equiv.) in water. Lastly, 10 ml of a 0.025M solution of $Pd(OAc)_2(Ph_3P)_2$ in DMF was added. The vials were agitated and heated to 75°C for 20 hours. After cooling down, each vial had 500 ml ethyl acetate added and was filtered through 250 mg of neutral alumina. The vials were dried in a vacuum oven (20 torr/40°C) equipped with a $N_2$ bleed. The vials were then diluted with 500 ml methanol and aliquots were removed to be analyzed by HPLC and MS. The vials were again dried *in vacuo,* treated with 1 ml of 2.5 M HCl/ethyl acetate for 3 hours at room temperature (RT). The vials were dried under a stream of $N_2$, followed by drying in a vacuum oven (20 torr/40°C). The vials were diluted in 500 ml methanol and agitated for 20 minutes to dissolve the samples. From each vial was drawn 50 ml to be dispensed onto a microtiter plate with matching 96 wells. Each vial also had an aliquot removed for HPLC and MS testing.

## EXAMPLE 2

2β-(3,5-DICHLOROBENZENE)-7-AZA-BICYCLO[2.2.1]HEPTANE, HCL SALT

MP 198.5-201.5°C.
IR: 2880.0, 2702.0, 2646.5, 2529.4, 1608.0, 1592.8, 1568.4, 1455.3, 1432.7, 1357.4, 1344.8, 892. 859.2, 798.5, 688.9 $cm^{-1}$.
MS: CI (m/z) 242.1/244.1 ($M+H^+$).
HRMS (m/z) 242.0509, calculated for $C_{12}H_{14}Cl_2N$, 242.0503.
$^1H$ NMR ($CDCl_3$) δ 9.79 (1H, br s), 9.29 (1H, br s), 7.35 (2H, s), 7.19 (1H, s), 4.36 (1H, br s), 4.22 (1H br s), 3.04

(1H br s), 2.31-2.20 (4H, br m), 1.70 (2H, br d J=47.6 Hz).
$^{13}C$ NMR ($CDCl_3$) δ 143.8, 135.4, 127.6, 126.2, 63.3, 58.5, 45.9, 36.9, 28.7 25.5 .

**EXAMPLE 3**

2β-(4-NITROPHENYL)-7-AZA-BICYCLO[2.2.1]HEPTANE, HCL SALT

MP 223.0-225.0°C.
IR: 2815.9, 2697.6, 2645.1, 2525.7, 1607.7, 1599.2, 1520.7, 1498.9, 1349.8, 1322.9, 1291.1, 887.3, 857.9, 842.7, 749.9, 701.1 $cm^{-1}$.
MS: CI (m/z) 219.1 ($M+H^+$).
HRMS (m/z), 219.1150, calculated for $C_{12}H_{15}N_2O_2$, 219.1134.
$^1H$ NMR ($CDCl_3$) δ 9.99 (1H, br s), 9.50 (1H, br s), 8.21 (2H, d J=8.5 Hz), 7.65 (2H, d, J=8.5 Hz), 4.40 (1H, s ), 4.20 (1H, s). 3.24 (1H, ap. t, J=8.7, 6.6 Hz), 2.36-2.24 (4H, m), 1.84 (1H, d, J=11.4 Hz), 1.73 (1H, ap.t, t, J=11.8, 6.4 Hz).
$^{13}C$ NMR ($CDCl_3$) δ 147.6, 147.1, 128.6, 124.2, 63.3, 58.6, 46.1, 37.0, 28.8, 25.6.

**EXAMPLE 4**

2β-(3-THIOPHENE)-7-AZA-BICYCLO[2.2.1]HEPTANE HCL SALT

MP 155-157.5°C.
IR: 2818.1, 2649.2, 2626.9, 2540.4, 1609.1, 1598.6, 1464.1, 1452.3, 1369.1, 1349.9, 1333.9, 884.9, 825.6, 786.7, 766.7 $cm^{-1}$.
MS: CI (m/z) 180.1 ($M+H^+$).
HRMS (m/z) 180.0863, calculated for $C_{10}H_{14}NS$, 180.0847.
$^1H$ NMR ($CDCl_3$) δ 9.99 (1H br s), 9.42 (1H br s), 7.46 (1H, s) 7.28 (1H, t, J=2.46 Hz) 7.13 (1H, d, J=4.91 Hz), 4.37 (1H, s), 4.02 (1H, d J=3.6 Hz), 3.20 (1H, dd, J=9.0, 6.0 Hz), 2.33-2.12 (4H, m), 1.77 (1H, ap. t, J=9.4 Hz, J=12.0 Hz), 1.63 (1H, ap. t, J=9.6, 9.0 Hz).

**EXAMPLE 5**

2β-(3-FLUORO-4-CHLOROPHENYL)-7-AZA-BICYCLO[2.2.1]HEPTANE, HCL SALT

MP 208.5-209.5°C.
IR: 2992.1, 2953.0, 2881.8, 2716.1, 2652.8, 2550.5, 1612.1, 1578.8, 1489.0, 1424.2, 1356.1, 1072.8, 884.4, 818.7, 535.5 $cm^{-1}$.
MS: CI (m/z) 226.0 ($M+H^+$).
$^1H$ NMR ($CDCl_3$) δ 9.9(1H br s), 9.4 (1H br s), 7.37 (1H, d, J=7.9 Hz), 7.25 (2H, m), 4.37 (1H, s), 4.11 (1H, d, J=3.5 Hz), 3.08 (1H, ap. t, J=6.8, 8.7 Hz), 2.34-2.29 (3H, m), 2.20 (1H, ap. t, J=9.3, 13.3 Hz), 1.81 (1H, ap. t, J=6.8, 11.8 Hz), 1.68 (1H, ap. t, J=12.2, 8.1 Hz).
$^{13}C$ NMR ($CDCl_3$) δ 141.4, 131.0, 123.9, 119.9, 116.2, 63.5, 58.6, 45.7, 36.9, 28.6, 25.5.

**EXAMPLE 6**

2β-(3-FLOUROPHENYL)-7-AZA-BICYCLO[2.2.1]HEPTANE, HCL SALT

MP 211.0-213.5°C.
IR: 2956.7, 2929.1, 2880.6, 2824.6, 2716.4, 2651.9, 2543.4, 2134.8, 1612.38. 1586.32, 1487.9, 1450.1, 1361.2, 1230.6, 1156.8, 893.9, 790.9, 775.4, 690.9 $cm^{-1}$.

MS: CI (m/z) 191.8 (M+H+).
HRMS (m/z), 192.1186, calculated for $C_{12}H_{15}NF$, 192.1189.
$^{1}H$ NMR ($CDCl_3$) δ 7.36-7.30 (2H, m), 7.13 (1H, d, J=9.3 Hz), 6.93 (1H, t, J=8.3, 6.6 Hz), 4.39 (1H, s), 4.10 (1H, d, J=3.4 Hz), 3.10 (1H, ap. t, J=9.0, 6.8 Hz), 2.35-2.32 (3H, m), 2.19 (1H, dd, J=13.6, 9.3 Hz), 1.80 (1H, ap. t, J=8.6, 12.0 Hz), 1.68 (1H, ap. t, J=11.3, 6.4 Hz).
$^{13}C$ NMR ($CDCl_3$), δ, 164.2, 142.5, 130.8, 130.7, 122.9, 115.0, 114.8, 114.5, 114.3 63.8, 58.5, 46.3, 37.1, 28.7, 25.5.

**EXAMPLE 7**

2β-(3-HYDROXYPHENYL)-7-AZA-BICYCLO[2.2.1]HEPTANE, HCL SALT

MP 222-224°C.
IR: 3214.5, 2897.6, 2717.7, 2644.0, 2542.7, 1618.2, 1605.1, 1587.9, 1494.7, 1465.8, 1378.1, 1357.3, 1337.0, 1324.9, 1302.9, 1281.7, 1273.9, 1166.5, 1157.2, 931.8, 851.4, 805.5, 780.8, 691.3, 670.2, 514.5, 448.8 $cm^{-1}$.
MS: CI (m/z) 190.1 (M+H+).
HRMS, 190.1249, calculated for $C_{12}H_{16}NO$, 190.1231.
$^{1}H$ NMR ($d_4$ $CD_3OD$) δ 7.16 (1H, t, J=7.9 Hz), 6.76-6.65 (3H, m), 4.40 (1H, d, J=2.9 Hz), 4.27 (1H, s), 2.34 (1H, dd, J=13.3, 9.5 Hz), 2.09-1.80 (6H, m).
$^{13}C$ NMR ($d_4$ $CD_3OD$) δ 157.7, 142.8, 129.6, 117.0, 113.5, 113.3, 63.0, 44.5, 36.3, 27.3, 25.5.

**EXAMPLE 8**

2β-(3-ACETOPHENONE)-7-AZA-BICYCLO[2.2.1]HEPTANE, HCL SALT

MP 181.5-183.8°C.
IR: 2996.1, 2962.5, 2840.8, 2791.3, 2697.9, 2639.3, 2528.5, 1678.5, 1602.9, 1581.5, 1362.6, 1295.4, 1279.9, 1260.0, 807.5, 702.3, 689.5 $cm^{-1}$.
MS: CI (m/z) 215.8 (M+H+).
HRMS, 216.1399, calculated for $C_{14}H_{18}NO$, 216.1388 .
$^{1}H$ NMR ($CDCl_3$) δ 9.79 (1H, br s), 9.18 (1H, br s), 7.87 (1H, s), 7.78 (1H, d, J=7.26 Hz), 7.69 (1H, d, J=5.77 Hz), 7.43 (1H, d, J=6.84 Hz), 4.38 (1H, br. s), 4.16 (1H, br. s), 3.19 (1H, br. s), 2.60 (3H, s), 2.30-2.20 (4H, m), 1.81 (1H, s), 1.67 (1H, s).
$^{13}C$ NMR ($CDCl_3$) δ 198.4, 141.0, 137.5, 132.1, 129.4, 127.5, 127.4, 63.8, 58.7, 46.2, 36.9, 28.8, 27.1, 25.6.

**EXAMPLE 9**

2β-(4-TRIFLUOROMETHYLPHENYL)-7-AZA-BICYCLO[2.2.1]HEPTANE, HCL SALT, OIL

IR: 2953.5, 2922.3, 2881.2, 2699.0, 2637.8, 2524.6, 1618.0, 1595.2, 1328.7, 1198.0, 1164.3, 1116.3, 1070.3, 1016.7, 887.8, 832.8 $cm^{-1}$.
MS: CI (m/z) 242.1 (M+H+).
HRMS (m/z) 242.1160, calculated for $C_{13}H_{15}F_3N$, 242.1156.
$^{1}H$ NMR ($CDCl_3$) δ 9.91 (1H, br s), 9.26 (1H, br s), 7.60 (1H, br s), 4.41 (1H, br s), 4.19 (1H br s), 3.81 (1H, br s), 2.35-2.24 (4H, br m), 1.84 (1H, br s), 1.71 (1H, br s). $^{13}C$ NMR ($CDCl_3$) d 128.1, 126.0, 64.0, 58.9, 46.5, 37.7, 29.1, 25.8.

EXAMPLE 10

2β-(3-FLUORO-4-METHYLPHENYL)-7-AZA-BICYCLO[2.2.1}HEPTANE HCL SALT

IR: 2879.9, 2822.4, 2690.1, 2650.8, 2543.9, 1609.5, 1577.7, 1508.1, 1372.0, 1352.3, 1326.7, 1274.4, 1251.5, 1118.2, 886.0, 816.4, 757.9, 520.0, 449.4 $cm^{-1}$.
MS: CI (m/z) 206.1 ($M+H^+$).
HRMS (m/z) 206.1357, calculated for $C_{13}H_{16}FN$, 206.1345.
$^1$H NMR ($CDCl_3$) δ 10.05 (1H br s), 9.2 (1H br s) 7.17 (2H, s), 7.04 (1H, d, J=10.7 Hz), 4.37 (1H, s), 4.08 (1H, s), 3.06 (1H, br s), 2.34 (4H, br s), 2.20 (3H, s), 1.79 (1H, s), 1.67 (1H, d, J=10.0 Hz).
$^{13}$C NMR ($CDCl_3$) δ 162.0, 160.0, 140.0, 132.1, 122.5, 114.6, 114.3, 63.9, 58.6, 45.9, 36.9, 28.7, 25.5 .

**EXAMPLE 11**

2β-(3-CHLOROPHENYL)-7-AZA-BICYCLO[2.2.1]HEPTANE HCL SALT

MP 187-189°C.
IR: 2929.5, 2895.2, 2854.2, 2712.2, 2688.0, 2650.5, 2544.7, 1610.5, 1596.0, 1569.9, 1480.7, 1464.4, 1454.6, 1434.3,1347.9, 901.4, 790.0, 696.2 $cm^{-1}$.
MS: CI (m/z) 207.7, 208.8, 209.7 ($M+H^+$).
HRMS (m/z) 208.0879, calculated for $C_{12}H_{14}ClN$, 208.0893.
$^1$H NMR ($CDCl_3$) δ 9.90 (1H, br s), 9.21 (1H, br s), 7.45 (1H, d J=7.47 Hz), 7.35-7.20 (3H, m), 4.38 (1H, s), 4.09 (1H, d, J=2.5 Hz), 3.07 (1H, t J=7.8 Hz), 2.34 (3H, br s), 2.18 (1H, dd, J=9.55 Hz, 13.3 Hz), 1.73 (2H, ap dt, J=48.6 Hz, 11.2 Hz, 7.9 Hz).
$^{13}$C NMR ($CDCl_3$) δ 142.5, 134.6, 130.5, 128.1, 127.6, 125.4, 63.7, 58.6, 46.1, 37.1, 28.8, 25.6.

**EXAMPLE 12**

2β-(N-BENZYL-5-PYRIDONYL)-7-AZA-BICYCLO[2.2.1]HEPTANE HCL SALT

MS: CI (m/z) 281.2 ($M+H^+$).
HRMS (m/z) 281.1661, calculated for $C_{18}H_{21}N_2O_2$, 281.1654.
$^1$H NMR ($CDCl_3$) δ 9.99 (1H, br s), 9.25 (1H, br s), 7.40-7.24 (7H, m), 6.74 (1H, br s), 5.47 (1H br s), 5.06 (1H, br s), 4.35 (1H, br s), 4.1 (1H, m), 2.65 (1H, br s), 2.31-2.03 (4H, m), 1.80 (1H, br s), 1.66 (1H, br s).

**EXAMPLE 13**

2β-(N-METHYL-5-PYRIDONYL)-7-AZA-BICYCLO[2.2.1]HEPTANE, HCL SALT

IR: 2995.0, 2953.2, 2810.6, 2643.2, 2530.7, 2156.8, 2129.1, 1671.0, 1644.5, 1607.7, 1594.8, 1534.7, 1449.1, 1438.5, 1373.7, 1357.7, 1346.5, 1322.4, 1312.9, 1292.2, 1256.5, 1196.5, 1180.2, 1161.7, 1150.4, 878.7, 835.3, 740.4, 529.0 $cm^{-1}$.
MS: CI (m/z) 205.1 ($M+H^+$).
HRMS (m/z) 205.1355, calculated for $C_{12}H_{17}N_2O$, 205.1341.
$^1$H NMR ($d_4$ $CD_3OD$) δ 8.34 (1H, s), 8.15 (1H, d, J=8.5 Hz), 7.17 (1H, d, J=8.7 Hz), 4.53 (1H, s), 4.36 (1H, s), 3.94 (3H, s), 3.44 (1H br s), 2.41 (1 H, ap t), 2.20-1.85 (5H, m).
$^{13}$C NMR ($d_4$ $CD_3OD$) δ 160.0, 159.0, 139.8, 129.3, 114.4, 62.4, 59.0, 41.9, 39.7, 35.2, 27.2, 25.5.

**EXAMPLE 14**

2β-(3-FLUORO-5-NITROPHENYL)-7-AZA-BICYCLO[2.2.1]HEPTANE, HCL SALT

MP 185.5-187.1°C.
IR: 2960.1, 2881.6, 2842.1, 2709.0, 2650.8, 2533.6, 1606.7, 1534.8, 1455.6, 1348.6, 1319.8, 1283.8, 1235.7, 1155.4, 899.5, 878.6, 870.2, 783.3, 747.7, 685.8 $cm^{-1}$.
MS: CI (m/z) 237.2 $(M+H^+)$.
HRMS (m/z) 237.1, calculated for $C_12H_{14}FN_2O_2$, 237.1039.
$^1H$ NMR ($CDCl_3$) δ 9.80 (1H, br s), 9.54 (1H, br s), 7.98 (1H, s), 7.76-7.71 (2H, m), 4.39 (2H, br s) 3.25 (1H, br s), 2.31 (4H, br s), 1.83 (1H, br s), 1.71 (1H, br s).
$^{13}C$ NMR ($CDCl_3$) δ 163.9, 161.9, 148.2, 144.6, 144.5, 121.2, 121.0, 118.8, 110.3, 110.1, 62.8, 58.6, 45.7, 37.0, 28.6, 25.6.

**EXAMPLE 15**

2β-(4-AMINOPHENYL)-7-AZA-BICYCLO[2.2.1]HEPTANE, HCL SALT

IR: 2874.2, 2566.6, 1975.0, 1598.1, 1572.8, 1512.8, 1468.1, 1344.2, 885.3, 818.2, 541.5, 499.3 $cm^{-1}$.
MS: CI (m/z) 189.1 $(M+H^+)$.
$^1H$ NMR ($d_4$ $CD_3OD$) δ 7.53 (2H, d, J=8.4 Hz), 7.43 (2H, d, J= 7.9 Hz), 4.5 (1H, d, J=2.9 Hz), 4.32 (1H, d, J=4.0 Hz), 3.45 (1H, dd, J=9.3, 6.0 Hz), 2.44 (1H, dd, J=13.2, 9.5 Hz), 2.12-1.85 (5H, m).
$^{13}C$ NMR ($d_4$ $CD_3OD$) δ 144.1, 130.7, 129.8, 124.7, 64.3, 60.5, 45.6, 37.8, 28.9, 26.8.

**EXAMPLE 16**

2β-(3-FLUORO-4-TRIFLUOROMETHYL-PHENYL)-7-AZA-BICYCLO[2.2.1]HEPTANE HCL SALT

MP 228.5-230.0°C.
IR: 2990.7, 2956.7, 2883.6, 2708.8, 2638.3, 2525.5, 1632.1, 1600.2, 1581.8, 1436.3, 1330.5, 1250.5, 1180.6, 1132.9. 1053.1, 834.8, 828.3 $cm^{-1}$.
MS: CI (m/z) 260.1 $(M+H^+)$.
$^1$HRMS (m/z), 260.1050. calculated for $C_{13}H_{14}F_4N$, 260.1062.
$^1H$ NMR ($CDCl_3$) δ 9.81 (1H, br s), 9.31 (1H, br s), 7.62 (1H, t, J=7.6 Hz), 7.44 (1H, d, J=8.1 Hz), 7.31 (1H, d, J=11.2 Hz), 4.40 (1H, s), 4.15 (1H, s), 3.16 (1H, t, J=7.5 Hz), 2.37-2.21 (4H, m), 1.77 (2H, dd, J=11.9, 41.2 Hz).
$^{13}C$ NMR ($CDCl_3$) δ 161.1, 158.5, 147.6, 147.5, 127.7, 127.7, 123.8, 123.3, 121.1, 116.4, 116.2, 63.3, 58.5, 45.9, 36.9, 28.8, 25.5.

**EXAMPLE 17**

2β-(4-CHLOROPHENYL)-7-AZA-BICYCLO[2.2.1]HEPTANE, HCL SALT

IR: 2956.6, 2879.7, 2815.7, 2690.7, 2646.7, 2541.2, 2138.5, 2114.9, 1609.4, 1600.4, 1494.3, 1465.1, 1454.3, 1371.6, 1349.8, 1326.4, 1095.0, 1014.2, 885.8, 824.4, 532.9, 504.5 $cm^{-1}$.
MS: CI (m/z), 208/210 $(M+H^+)$.
$^1H$ NMR, 250 MHz ($d_6$ DMSO) d 9.0 (2H, br s), 7.40 (4H, s), 4.36 (1H, d, J=3.2Hz), 4.19 (1H br s), 3.26 (1H, dd, J=9.3, 6.4 Hz), 2.28 (1H, dd, J=12.9, 9.6 Hz), 1.99-1.59 (5H, m).
$^{13}C$ NMR ($d_6$ DMSO) δ 138.9, 133.2, 129.0, 128.9, 63.8, 58.6, 45.8, 36.9, 28.6, 25.5.

**Example 18**

2β-(3,4-METHYLENEDIOXYPHENYL)-7-AZA-BICYCLO[2.2.1]HEPTANE, HCL SALT

MP 220.0-221.5°C.
IR: 2959.7, 2888.7, 2819.2, 2714.1, 2687.6, 2649.0, 2541.7, 1608.6, 1503.4, 1490.3, 1441.7, 1369.6, 1264.0, 1235.3, 1040.5, 930.0, 806.9, 548.4, 524.6, 419.8 $cm^{-1}$.
MS: CI (m/z) 218 ($M+H^+$).
HRMS (m/z) 218.1185, calculated for $C_{13}H_{16}NO_2$, 218.1181.
$^1H$ NMR, 250 MHz ($d_6$ DMSO) δ 7.03 (1H, d, J=1.6 Hz), 6.86 (1H, d, J=8.0 Hz), 6.79 (1H, dd, J=8.1, 1.6 Hz), 5.98 (2H, s), 4.25 (1H, d, J=2.9 Hz), 4.16 (1H, s), 3.16 (1H, dd, J=9.2, 6.3 Hz), 2.21 (1H, dd, J=13.0, 9.5 Hz), 1.92-1.61 (5H, m).
$^{13}C$ NMR, 250 MHz (d6 DMSO) δ 147.5, 145.8, 136.0, 120.3, 108.1, 107.8, 100.9, 62.4, 57.9, 44.3, 27.8, 25.2.

**EXAMPLE 19**

2β-(2-CHLORO-6-METHYL-5-PYRIDINYL)-7-AZA-BICYCLO[2.2.1]HEPTANE, HCL SALT

MS CI (m/z) 223, 225 ($M+H^+$).
HRMS (m/z), 223.1011, calculated for $C_{12}H_{16}ClN_2$, 223.1002.
$^1H$ NMR ($CDCl_3$) δ 7.73 (1H, d, J=8.1 Hz), 7.29 (1H, d, J=8.1 Hz), 4.51 (1H, d, J=3.7 Hz), 4.29 (1H, s), 3.50-3.45 (1H, m), 2.58-2.46 (4H, m), 2.08-1 82 (5H, m)

**EXAMPLE 20**

2β-(4-CYANOPHENYL)-7-AZA-BICYCLO[2.2.1]HEPTANE, HCL SALT

IR: 2938.1, 2878.9, 2858.6, 2831.9, 2741.6, 2721.7, 2693.8, 2649.2, 2556.1, 2532.8, 2230.0, 1609.6, 1508.7, 1376.9, 1349.4, 1327.8, 1301.8, 1182.4, 886.3, 847.9, 837.6, 553.1, 550.4, 537.5 $cm^{-1}$.
MS: CI (m/z) 199.1 ($M+H^+$).
HRMS (m/z) 199.1255, calculated for $C_{13}H_{15}N_2$, 199.1235.
$^1H$ NMR ($d_4$ $CD_3OD$) δ 7.72 (2H, d, J=8.3 Hz), 7.53 (2H, d, J=8.1 Hz), 4.55 (1H, d, J=3.2 Hz), 4.32 (1H, d, J=4.1), 3.47 (1H, dd, , J=9.2, 6.0 Hz). 2.45 (1H, dd, J=13.2, 9.6 Hz), 2.14-1.80 (5H, m).
$^{13}C$ NMR ($d_4$ $CD_3OD$) δ 146.9, 132.4, 127.7, 138.1, 110.5, 62.5, 59.0, 44.7, 36.3, 27.3, 25.4.

**EXAMPLE 21**

2β-(3-FLUORO-4-NITRO-PHENYL)-7-AZA-BICYCLO[2.2.1]HEPTANE, HCL SALT

MP 186.5-188.0°C.
IR: 2955.5, 2881.6, 2851.3, 2694.6, 2641.9, 2545.6, 1612.4, 1600.8, 1524.9, 1511.8, 1345.9, 1325.7, 1248.6, 1061.9, 939.1, 888.7, 833.8, 751.1, 578.2, 547.7, 537.3, 526.1 $cm^{-1}$.
MS: CI (m/z) 237.1 ($M+H^+$).
HRMS (m/z), 237.1023, calculated for $C_{12}H_{14}FN_2O_2$, 237.1039.
$^1H$ NMR ($d_4$ $CD_3OD$) δ 8.09 (1H, t, J=8.1 Hz), 7.46 (1H, d, J=12.5 Hz), 7.36 (1H, d, J=8.1 Hz), 4.59 (1H, s), 4.33 (1H, s), 3.51 (1H, d, J=5.9 Hz), 2.48 (1H, ap. t, J=12.8, 9.9 Hz), 2.07-1.86 (5H, m).
$^{13}C$ NMR ($d_4$ $CD_3OD$) δ 156.8, 154.1, 149.5,136.0, 126.6, 123.8, 117.9, 117.6, 62.9, 58.5, 45.8, 36.8, 28.6, 25.4.

**EXAMPLE 22**

2β-(4-AMIDO-PHENYL)-7-AZA-BICYCLO[2.2.1]HEPTANE, HCL SALT

MP 251.5-253.0°C.
IR: 3363.9, 3160.4, 2989.7, 2950.3, 2879.0, 2856.1, 2782.2, 2701.3, 2652.7, 2638.0, 2524.9, 1671.3, 1658.2, 1623.0, 1611.4, 1599.3, 1560.0, 1416.7, 1398.2, 1374.0, 888.1, 850.7, 778.0, 760.0, 747.4, 625.6, 606.7, 532.2, 473.2 $cm^{-1}$.
MS: CI (m/z), 217.1 ($M+H^+$).
$^1$H NMR ($d_4$ $CD_3OD$) δ 7.87 (2H, d, J=8.5 Hz), 7.41 (2H, d, J=8.1 Hz), 4.52 (1H, s), 4.29 (1H, s), 3.45 (1H, ap. t, J=6.0, 3.3 Hz), 2.42 (1H, ap. t, J=9.8, 3.5 Hz), 2.05-1.88 (5H, m).
$^{13}$C NMR ($d_4$ $CD_3OD$) δ 172.1, 147.2, 133.1, 129.5, 128.2, 64.2, 60.5, 46.0, 37.8, 28.9, 26.9.

**EXAMPLE 23**

2β-(3-FLUORO-4-AMINO-PHENYL)-7-AZA-BICYCLO[2.2.1]HEPTANE, HCL SALT

MP 266.0-270.0 °C.
IR: 2988.3, 2819.9, 2639.1, 2539.0, 2001.5, 1608.2, 1598.5, 1568.9, 1555.2, 1510.1, 1424.3, 1369.6, 1340.9, 1268.8, 1254.8, 893.5, 884.2, 837.4, 470.5, 452.6 $cm^{-1}$.
MS: CI (m/z) 207.1 ($M+H^+$).
HRMS (m/z) 207.1290, calculated for $C_{12}H_{16}FN_2$, 207.1297.
$^1$H NMR ($d_4$ $CD_3OD$) δ 7.46-7.39 (2H, m), 7.29 (1H, d, J=8.4 Hz), 4.51 (1H, s), 4.3 (1H, s), 3.44 (1H, dd, J=9.5, 5.9 Hz), 2.43 (1H, dd, J=13.2, 9.9 Hz), 2.09-1.86 (5H, m).

**Example 24**

2β-(4-SULFONAMIDO-PHENYL)-7-AZA-BICYCLO[2.2.1]HEPTANE, HCL SALT

MP 245.5-247.0°C.
IR: 3223.5, 3188.5, 3024.0, 2956.7, 2862.6, 2826.1, 2695.1, 2646.9, 2531.0, 1607.4, 1327.6, 1152.3, 1099.1, 912.1, 888.4, 832.5, 679.2, 617.2, 579.0, 558.4, 548.0, 516.9 $cm^{-1}$.
MS: CI (m/z) 253.1.
$^1$H NMR ($d_4$ $CD_3OD$) δ 7.88 (2H, d, J=8.4 Hz), 7.51 (2H, d, J=8.4 Hz), 4.55 (1H, d, J=3.0 Hz), 4.31 (1H, d, J=4.0 Hz), 3.48 (1H, dd, J=9.2, 6.2 Hz), 2.45 (1H, dd, J=13.4, 9.7 Hz), 2.11-1.86(5H, m).

**EXAMPLE 25**

2β-(3-METHYL-5-ISOXZAZOLE)-7-AZA-BICYCLO[2.2.1]HEPTANE, HCL SALT

MP 172.5-178.0°C.
IR: 2959.2, 2842.3, 2802.6, 2705.7, 2691.1, 2666.7, 2639.7, 2529.7, 1606.0, 1465.6, 1442.0, 1415.0, 1374.2, 1355.3, 1149.3, 890.9, 887.7, 824.0, 529.4 $cm^{-1}$.
MS: CI (m/z), 179 ($M+H^+$).
HRMS, 179, 1177, calculated for $C_{10}H_{14}N_2O$, 179. 1184.
$^1$H NMR ($CDCl_3$) δ 10.11 (1H, br s), 9.19 (1H, s), 6.42 (1H, s), 4.38 (1H, s), 4.24 (1H, s), 3.26 (1H, ap. t, J=8.3, 6.23 Hz), 2.32-2.16 (7H, m), 1.74 (2H, dd, J=29.4, 10.8 Hz).
$^{13}$C NMR ($CDCl_3$) δ 170.7, 160.2, 103.5, 63.1, 62.0, 58.2, 38.5, 36.8, 35.1, 27.7, 25.4, 11.5.

**EXAMPLE 26**

2β-(3-METHYL-5-ISOXZAZOLE)-7-AZA-BICYCLO[2.2.1]HEPTANE, N-METHYL

MS CI (m/z), 193 ($M+H^+$).
$^1H$ NMR ($CDCl_3$) δ 5.91 (1H, s), 3.46 (1H, d, J=3.7 Hz), 3.37 (1H, t, J=4.2 Hz), 2.87 (1H, dd, J=9.0, 5.1 Hz), 2.26 (3H, s), 2.25 (3H, s), 1.98-1.83 (4H, m), 1.53-1.39 (2H, m).

**EXAMPLE 27**

2β-(3-METHYL-5-ISOXZAZOLE)-7-AZA-BICYCLO[2.2.1]HEPTANE, N-ACETYL

MS: CI (m/z), 221 ($M+H^+$), 238 ($M+NH_4^+$).
$^{13}C$ NMR ($CDCl_3$) δ 174.6, 174.4, 167.5, 166.8, 159.8, 101.9, 100.6, 61.0, 56.8, 56.4, 52.6, 41.6, 40.1, 38.4, 36.1, 29.9, 28.4, 28.3, 21.4, 11.4.

**EXAMPLE 28**

2B-(3,4-DIFLUOROPHENYL)-7-AZABICYCLO[2.2.1]HEPTANE, HCL SALT

MP 206.5-208.5°C.
IR: (KBr), 2992.7, 2953.8, 2929.1, 2882.0, 2827.2, 2717.0, 2653.3, 2547.4, 1434.1, 1373.1, 1358.9, 1281.1, 1121.1, 888.2, 823.1, 763.4 $cm^{-1}$.
MS: CI (m/z) 210 ($M+H^+$).
HRMS (m/z) 210.1102, calculated for $C_{12}H_{14}NF_2$, 210.1094.
$^1H$ NMR ($CDCl_3$) δ 9.91(1H, br s), 9.32 (1H, br s), 7.32-7.12 (3H, m), 4.39 (1H, s), 4.08 (1H, d, J=3.5 Hz), 3.1 (1H, dd, J=8.8, 6.7 Hz), 2.35-2.17 (4H, m), 1.81 (1H, ap. t, J=7.1, 11.6 Hz), 1.7 (1H, ap. t, J=11.8, 8.5 Hz).
$^{13}C$ NMR ($CDCl_3$) δ 137.5, 123.4, 117.8, 117.6, 117.1, 116.8, 63.8, 58.5, 45.7, 37.1, 28.7, 25.5.

**EXAMPLE 29**

4-(7-AZA-BICYCLO[2.2.1]HEPT-2-YL)-BENZAMIDINE HCL SALT

MP 198.5-201.0°C.
IR: (KBr), 3031.3, 2911.0, 2844.1, 2707.5, 2643.8, 2527.3, 1677.2, 1612.3, 1600.4, 1480.5, 1470.6, 1446.3, 1409.7, 1366.1, 1343.0, 1324.9, 1159.8, 886.2, 833.1, 756.3, 738.1, 684.1, 634.7, 528.0 $cm^{-1}$.
MS: CI (m/z) 216.2 ($M+H^+$).
HRMS (m/z), 216.1505, calculated for $C_{13}H_{18}N_3$, 216.1501.
$^1H$ NMR (d4 CD40D) δ 7.82 (2H, d, J=8.1 Hz), 7.59 (2H, d, J=8.3 Hz), 4.56 (1H, d, J=3.7 Hz), 4.33 (1H, br. s), 3.51 (1H, dd, J=6.0, 9.5 Hz), 2.47 (1H, dd, J=13.4, 9.5 Hz), 2.08-1.89 (5H, m).
$^{13}C$ NMR ($d_4$, $CD_4OD$) δ 149.7, 129.6, 129.0, 64.0, 60.4, 46.0, 37.8, 28.9, 26.8 .

**EXAMPLE 30**

2-(4-METHANESULFONYL-PHENYL)-7-AZA-BICYCLO[2.2.1]HEPTANE, HCL SALT

MP 242.5-244.0°C.
IR: (KBr), 3015.1, 2993.2, 2949.8, 2929.8, 2874.1, 2812.3, 2701.8, 2644.7, 2531.6, 1360.7, 1597.8, 1360.7,

1324.3, 1302.8, 1289.8, 1169.3, 1146.6, 1087.6, 954.0, 826.9, 776.7, 560.7, 534.6, 523.6, 488.1 $cm^{-1}$.
MS: CI (m/z) 252.1 ($M+H^+$).
HRMS (m/z), 252.1081, calculated $C_{13}H_{18}NOS$, 252.1058.
$^1H$ NMR ($d_4$ $CD_3OD$) δ 7.94 (2H, d, J=8.3 Hz), 7.59 (2H, d, J=8.3 Hz), 4.57 (1H, d, J=3.7 Hz), 4.32 (1H, br. s), 3.51 (1H, dd, J=9.3, 5.8 Hz), 3.10 (3H, s), 2.47 (1H, dd, J=13.4, 9.7 Hz), 2.11-1.68 (5H, m).
$^{13}C$ NMR ($d_4CD_3OD$) δ 149.2, 140.8, 129.1, 129.0, 64.0, 60.5, 46.0, 44.4, 37.8, 28.8, 26.8.

**EXAMPLE 31**

4-(7-AZA-BICYCLO[2.2.1]HEPT-2-YL)-PHENOL HCL SALT

MP 242.5-244.0°C.
IR: (KBr), 3162.0, 3106.4, 3010.9, 2982.8, 2967.3, 2953.9, 2881.1, 2830.4, 2697.8, 2657.5, 2577.4, 2530.5, 1614.2, 1605.2, 1589.5, 1518.1, 1460.6, 1448.1, 1439.6, 1355.5, 1333.0, 1308.4, 1268.9, 1252.1, 1242.1, 1229.9, 1191.7, 1162.2, 1154.1, 892.7, 840.0, 828.1, 706.8, 509.9 $cm^{-1}$.
MS: CI (m/z) 190.2 ($M+H^+$); HRMS (m/z), 190.1214, calculated for $C_{12}H_{16}NO$, 190.1232.
$^1H$ NMR ($d_4$ $CD_3OD$) δ 7.11 (2H, d, J=8.0 Hz), 6.77 (2H, d, J=7.7 Hz), 4.34 (1H, d, J=2.3Hz), 4.27 (1H, br s), 2.32 (1H, dd J=13.4, 9.4 Hz), 2.11-1.82 (5H, m).
$^{13}C$ NMR ($d_4$ $CD_3OD$) δ 156.2, 131.8, 130.7, 127.4, 115.2, 63.6, 59.0, 44.1, 36.2, 27.3, 25.5.

**EXAMPLE 32**

2-(4-METHYLSULFANYL-PHENYL)-7-AZA-BICYCLO[2.2.1]HEPTANE HCL SALT

MP 216.5-218.0°C.
IR: (KBr), 3021.7, 2992.9, 2979.5, 2958.1, 2874.2, 2853.7, 2821.8, 2716.1, 2689.7, 2651.6, 2550.7, 2535.3, 2138.4, 1609.4, 1497.2, 1465.0, 1453.2, 1439.2, 1427.5, 1371.6, 1355.0, 1327.1, 1095.8, 1016.4, 974.8, 887.7, 820.4, 790.2, 534.1, 506.0 $cm^{-1}$.
MS:CI (m/z) 220.2 ($M+H^+$).
HRMS (m/z), 220.1174, calculated for $C_{13}H_{18}NS$, 220.1160.
$^1H$ NMR ($d_4CD_3OD$) δ 7.28-7.22 (4H, m), 4.41 (1H, d, J=2.3 Hz), 4.29 (1H, br. s), 3.33 (1H, dd, J=9.1, 5.8 Hz), 2.44 (3H, s), (1H, dd, J=13.0, 9.6 Hz), 2.10-1.83 (5H, m).
$^{13}C$ NMR ($d_4$ $CD_3OD$) δ 128.0, 127.6, 64.2, 60.2, 45.1, 37.9, 28.8, 26.8.

**EXAMPLE 33**

4-(7-AZA-BICYCLO[2.2.1]HEPT-2-YL)-BENZOIC ACID METHYL ESTER HCL SALT

IR: (KBr), 2995.9, 2983.0, 2959.8, 2906.0, 2882.8, 2850.0, 2812.8, 2713.2, 2686.8, 2649.6, 2622.6, 2533.5, 1726.3, 1608.0, 1464.0, 1457.6, 1436.7, 1417.9, 1371.4, 1348.7, 1326.6, 1279.5, 1191.7, 1140.6, 1106.2, 1018.5, 959.0, 892.0, 842.7, 776.0, 761.6, 705.9, 536.0, 511.2 $cm^{-1}$.
MP: 235.0-236.0C.
MS: CI (m/z) 232.2 ($M+H^+$).
HRMS (m/z), 232.1348, calculated for $C_{14}H_{18}NO_2$, 232.1337.
$^1H$ NMR ($d_4$ $CD_3OD$) δ 8.00 (2H, d, J=8.1 Hz), 7.43 (2H, d, J=8.5 Hz), 4.53 (1H, d, J=7.1 Hz), 4.29 (1H. s), 3.88 (3H, s), 3.48-3.44 (1H, m), 2.44 (1H, dd, J=13.3, 9.8 Hz), 2.11-1.85 (5H, m).
$^{13}C$ NMR ($CDCl_3$) δ 166.8, 145.4, 130.2, 129.0, 127.5, 63.4, 58.5, 52.0, 46.3, 36.9, 28.7, 25.5.

**EXAMPLE 34**

4-(7-AZA-BICYCLO[2.2.1]HEPT-2-YL)-BENZOIC ACID HCL SALT

MP 261.5-264.5 °C.
IR: (KBr), 3090.6, 3038.8, 2980.8, 2956.9, 2932.8, 2884.7, 2699.0, 26415, 2576.3, 2507.9, 1682.2, 1607.3, 1573.6, 1467.4, 1421.9, 1403.3, 1371.6, 1354.1, 1322.2, 1308.7, 1296.2, 1264.2, 1222.7, 1155.5, 1125.8, 1112.9, 887.9, 850.8, 830.5, 776.5, 766.4, 711.2, 696.1, 529.4, 506.7 $cm^{-1}$.
MS: Cl (m/z) 218.2 ($M+H^+$).
HRMS (m/z) 218.1181, calculated for $C_{13}H_{16}NO_2$, 218.1181.
$^1$H NMR ($d_4$ $CD_3OD$) δ 8.01 (2H, d, J=8.1 Hz), 7.42 (2H, d, J=8.5 Hz), 4.54 (1H, d, J=2.9 Hz), 4.30 (1H, s), 3.46 (1H, dd, J=9.2, 6.3 Hz), 2.44 (1H, dd, J=13.4, 9.6 Hz), 2.12-1.86 (5H, m).
$^{13}$C NMR ($d_4$ $CD_3OD$) δ 168.5, 146.9, 130.2, 126.9, 63.0, 59.3, 44.9, 36.7, 27.7, 25.8.

**EXAMPLE 35**

2-(3-FLUORO-4-TETRAZOL-1-YL-PHENYL)-7-AZA-BICYCLO[2.2.1]HEPTANE HCL SALT

MP: decomposes 231°C dec.
IR: (KBr), 3082.8, 3012.2, 2988.2, 2963.7, 2941.3, 2881.4, 2842.4, 2826.9, 2803.8, 2720.2, 2706.0, 2659.9, 2640.8, 2540.5, 2529.3, 2493.5, 2382.8, 1603.4, 1527.5, 1465.4, 1453.8, 1402.7, 1373.0, 1239.7, 1214.9, 1172.6, 1146.9, 1085.9, 993.5, 897.4, 830.5, 622.8, 540.2, 523.7, 404.3 $cm^{-1}$.
MS: Cl (m/z) 260.3 ($M+H^+$).
HRMS (m/z), 260.1317, calculated for $C_{13}H_{15}N_5F$, 260.1311.
$^1$H NMR ($d_4$ $CD_3OD$) δ 9.61 (1H, d, J=1.7 Hz), 7.87 (1H, t, J=8.1Hz), 7.52 (1H, d, J=11.8 Hz), 7.41 (1H, d, J=8.3 Hz), 4.57 (1H, d, J=3.5 Hz), 4.32 (1H, s), 3.52 (1H, dd, J=9.2, 6.1 Hz), 2.48 (1H, dd, J=13.4, 9.6 Hz), 2.13-1.86 (5H, m).
$^{13}$C NMR ($d_4$ $CD_3OD$) δ 156.0, 153.5, 143.9, 143.8, 125.8, 124.2, 115.9, 115.7, 62.9, 59.3, 44.5, 36.6, 27.6, 25.7.

**EXAMPLE 36**

2-(4-NITRO-3-TRIFLUOROMETHYL-PHENYL)-7-AZA-BICYCLO[2.2.1]HEPTANE HCL SALT

IR: (KBr), 2956.9, 2882.3, 2814.2, 2707.1, 2642.7, 2531.1, 1602.6, 1539.4, 1495.2, 1469.2, 1454.3, 1421.1, 1362.0, 1323.6, 1282.8, 1212.2, 1177.9, 1142.9, 1048.3, 906.5, 869, 857.4, 841.8, 822.6 $cm^{-1}$.
MS: Cl (m/z), 287.2 ($M+H^+$).
HRMS (m/z) 287.1016, calculated for $C_{13}H_{14}F_3N_2O_2$, 287.1007.
$^1$H NMR ($CDCl_3$) δ 10.15 (1H, br. s), 9.79 (1H, br. s), 8.11 (1H, d, J=8.3 Hz), 7.95 (1H, d, J=8.3 Hz), 7.72 (1H, s), 4.42 (1H, br. s), 4.20 (1H, br. s), 3.29 (1H, ap. t, J=8.5, 7.3 Hz), 2.37-2.29 (4H, m), (2H, dd, J=45.8, 10.3 Hz).
$^{13}$C NMR ($CDCl_3$) δ 146.0, 145.7, 131.9, 127.93, 127.89, 126.0, 123.6, 63.1, 58.4, 46.0, 36.8, 28.7, 25.4.

**EXAMPLE 37**

2-[3-FLUORO-4-(5-TRIFLUOROMETHYL-TETRAZOL-1-YL)-PHENYL]-7-AZA-BICYCLO[2.2.1]HEPTANE, HCL SALT

MP 195.5-198.5°C.
IR (KBr), 2988.4, 2955.7, 2882.2, 2703.2, 2639.7, 2529.1, 1620.3, 1603.3, 1538.7, 1517.9, 1469.5, 1452.9, 1436.9, 1358.1, 1322.5, 1279.8, 1247.2, 1220.4, 1173.7, 1136.8, 1106.5, 1059.3, 1037.7, 1016.9, 982.0, 937.0, 883.9, 830.4, 823.9, 772.4, 757.5, 638.6, 532.0, 497.3 $cm^{-1}$.

MS: CI (m/z) 328.1 (M+H+).

HRMS (m/z) 328.1185, calculated for $C_{14}H_{14}N_5F_4$, 328.1185.

$^1$H NMR ($d_4$ $CD_3OD$) δ 7.74 (1H, t, J=8.0), 7.59 (1H, dd, J=11.2, 1.7 Hz), 7.49 (1H, dd, J=8.3, 0.8 Hz), 4.63 (1H, d, J=3.7 Hz), 4.34 (1H, ap. t, J=4.4, 3.7), 3.58 (1H, dd, J=9.5, 6.1 Hz), 2.52 (1H, dd, J=13.4, 9.7 Hz), 2.17-1.88 (5H,m).

$^{13}$C NMR ($CDCl_3$) δ 175.7, 157.5, 154.9, 147.8, 128.4, 124.6, 119.4, 119.3, 117.0, 116.8, 63.4, 58.7, 46.1, 37.1, 28.8, 25.4.

**EXAMPLE 38**

2-(3-CHLORO-4-NITRO-PHENYL)-7-AZA-BICYCLO[2.2.1]HEPTANE, HCL SALT

MP 242.5-244.5°C.

IR: (KBr), 3104.1, 3040.4, 3020.0, 2995.1, 2961.2, 2863.3, 2842.6, 2794.1, 2685.3, 2642.8, 2609.5, 2587.1, 2575.8, 2526.9, 2384.2, 1609.0, 1593.6, 1584.2, 1520.0, 1478.5, 1466.4, 1342.8, 1322.9, 1303.4, 1292.1, 1280.1, 1271.8, 1254.3. 1234.3, 1214.5, 1165.0, 1139.3, 1060.0, 1049.0, 930.8, 905.1, 882.0, 865.3, 842.5, 816.7, 750.0, 704.9, 693.2, 531.5, 447.1 $cm^{-1}$.

MS: CI (M+H+) m/z=253.1/255.1.

HRMS (m/z) 253.0741, calculated for $C_{12}H_{13}ClN_2O_2$, 253.0744.

$^1$H NMR ($d_4$ $CD_3OD$) δ 7.93 (1H, d, J=8.5 Hz), 7.67 (1H, d, J=1.7 Hz), 7.47 (1H, dd, J=8.5, 1.9 Hz), 4.57 (1H, d, J=3.5 Hz), 4.31 (1H, ap. t, J=3.9, 4.4 Hz), 3.49 (1H, dd, J=9.5, 6.2 Hz), 2.46 (1H. dd, J=9.8, 13.5 Hz), 2.07-1.85 (5H, m).

$^{13}$C NMR ($d_4$ $CD_3OD$) δ 148.1, 130.2, 126.7, 125.9, 62.6, 59.2, 44.4, 36.6, 27.6, 25.6.

**EXAMPLE 39**

2-(4-TETRAZOL-1-YL-PHENYL)-7-AZA-BICYCLO[2.2.1]HEPTANE, HCL SALT

IR: (KBr), 3070.4, 2967.1, 2952.1, 2914.5, 2877.0, 2745.9, 2711.5, 2673.5, 2650.7, 2547.0, 1601.5, 1524.0, 1469.8, 1398.0, 1374.9, 1362.7, 1346.7, 1328.7, 1322.5, 1313.2, 1252.1, 1217.1, 1193.9, 1183.3, 1091.6, 1057.2, 1041.8, 995.4, 907.7, 890.2, 856.7, 834.6, 812.6, 538.2, 522.2 $cm^{-1}$.

MS: CI (M+H+) m/z=242.1.

HRMS (m/z) 242.1421, calculated for $C_{13}H_{16}N_5$, 242.1406.

$^1$H NMR ($d_4$ $CD_3OD$) δ 9.77 (1H, s), 7.88 (2H, d, J=8.1 Hz), 7.60 (2H, d, J=8.1 Hz), 4.57 (1H, br. s), 4.34 (1H, br. s), 3.51 (1H, ap. t, J=8.5, 6.4 Hz), 2.48 (1H, dd, J=9.9, 13.0 Hz), 2.16-1.88 (5H, m).

**EXAMPLE 40**

2-(6-METHOXY-PYRIDIN-2-YL)-7-AZA-BICYCLO[2.2.1]HEPTANE, HCL SALT

MS CI (M+H+), (m/z)=205.1.

HRMS (m/z) 205.1343, calculated for $C_{12}H_{17}N_2O$, 205.1341.

$^1$H NMR ($d_4$ $CD_3OD$) δ 7.62 (1H, t, J=7.8 Hz), 6.85 (1H, d, J=7.3 Hz), 6.69 (1H, d, J=8.3 Hz), 4.36 (2H, m), 3.36 (1H, dd, J=9.2, 4.0 Hz), 2.30 (1H, dd, J=13.3, 9.5 Hz), 2.06-1.85 (5H, m).

$^{13}$C NMR ($d_4$ $CD_3OD$) δ 145.5, 115.4, 109.3, 62.8, 58.9, 56.0, 44.1, 35.4, 26.8, 26.0.

**EXAMPLE 41**

2-(4-METHANESULFINYL-PHENYL)-7-AZA-BICYCLO[2.2.1]HEPTANE, HCL SALT

IR: (KBr), 2978.6, 2952.2, 2883.7, 2840.5, 2700.9, 2643.4, 2528.2, 1688.8, 1601.1, 1497.0, 1466.8, 1413.9, 1366.3, 1325.4, 1297.8, 1220.1, 1199.4, 1173.9, 1159.1, 1089.8, 1046.1, 1012.1, 956.6, 888.0, 826.0, 538.8, 519.6, 480.2 $cm^{-1}$.
MS: CI ($M+H^+$), m/z=236.1.
HRMS (m/z) 236.1103, calculated for $C_{13}H_{18}NOS$, 236.1109.
$^1H$ NMR ($CDCl_3$) $\delta$ 7.66 (4H, br s), 4.42 (1H, br s), 4.14 (1H, br s), 3.20 (1H br s), 2.71 (3H, s), 2.39-2.26 (4H, m), 1.87-1.73 (2H, m).
$^{13}C$ NMR ($d_4$ $CD_3OD$) $\delta$ 145.5, 143.9, 128.1, 124.4, 63.1, 59.3, 44.8, 42.7, 36.7, 27.7, 25.8.

**EXAMPLE 42**

2-(4-BROMO-3-FLUORO-PHENYL)-7-AZA-BICYCLO[2.2.1]HEPTANE, HCL SALT

MP 195.7-197.3°C.
IR (KBr): 2992.5, 2956.9, 2879.5, 2858.3, 2823.7, 2714.2, 2690.3, 2651.1, 2544.8, 1610.2. 1587.8, 1577.0, 1486.4, 1465.1. 1454.6, 1419.4, 1372.0, 1353.3, 1327.0, 1305.9, 1279.4. 1242.2, 1230.6, 1170.6, 1154.0, 1067.8, 1042.7, 982.7, 884.0, 812.5, 773.5, 767.6, 695.2, 547.4, 532.0 $cm^{-1}$.
MS: CI ($M+H^+$), m/z=270.1/272.0.
HRMS (m/z) 270.0298, calculated for $C_{12}H_{14}BrFN$, 270.0293.
$^1H$ NMR ($d_4$ $CD_3OD$) $\delta$ 7.60 (1H, t, J=7.8 Hz), 7.24 (1H, d, 9.8 Hz), 7.07 (1H, d, J=8.5 Hz), 4.48 (1H, br s), 4.28 (1H, br s), 3.38 (1H, ap. t, J=5.8, 9.3 Hz), 2.41 (1H, dd, J=13.4, 9.9 Hz), 2.04-1.83 (5H, m).

**EXAMPLE 43**

2-(4-CYANO-3-FLUORO-PHENYL)-7-AZA-BICYCLO[2.2.1]HEPTANE, HCL SALT

MP 98.7-99.8°C.
IR: (KBr) 3086.8, 3063.6, 3034.4, 2998.3, 2987.6, 2957.3, 2883.0, 2844.3, 2810.8, 2735.6, 2707.1, 2669.7, 2642.7, 2529.7, 2235.3, 1623.1, 1601.9, 1568.1, 1507.0, 1467.4, 1451.0, 1433.7, 1373.4, 1356.8, 1326.9, 1314.9, 1297.6, 1262.7, 1252.9, 1228.4, 1183.7, 1165.2, 1153.2, 1116.9, 1060.9, 938.3, 891.9, 824.1, 809.0, 736.4, 521.1, 506.5 $cm^{-1}$.
MS: CI ($M+H^+$), m/z=217.
HRMS (m/z) 217.1158, calculated for $C_{13}H_{14}FN_2$, 217.1141.
$^1H$ NMR ($d_4$ $CD_3OD$) $\delta$ 7.74 (1H, t, J=7.6 Hz), 7.39 (1H, d, J=10.8 Hz), 7.32 (1H, dd, J=8.1, 1.7 Hz), 4.55 (1H, d, J=3.5 Hz), 4.30 (1H, t, J=3.9 Hz), 3.49 (1H, dd, J=9.6, 6.0 Hz), 2.45 (1H, dd, J=13.3, 9.8 Hz), 2.07-1.84 (5H, m).

**EXAMPLE 44**

2-(3,4,5-TRIFLUORO-PHENYL)-7-AZA-BICYCLO[2.2.1]HEPTANE, HCL SALT.

MP 186.0-189.0°C.
IR: (KBr) 3014.2, 2963.0, 2866.0, 2831.6, 2699.0, 2646.3, 2614.4, 2584.9, 2537.7, 1619.2, 1609.6, 1532.9, 1458.1, 1447.2, 1373.3, 1352.6, 1321.5, 1312.8, 1275.2, 1240.4, 1224.5, 1173.5, 1158.0, 1068.6, 1041.4, 1020.8, 895.7, 881.8, 846.3, 789.3, 733.9, 533.6 $cm^{-1}$.
MS: CI ($M+H^+$) 228.
HRMS (m/z) 228.1004, calculated for $C_{12}H_{13}F_3N$, 228.1000.

$^1H$ NMR ($CDCl_3$) δ 9.99 (1H, br s), 9.54 (1H, br s), 7.17 (2H, t, J=7.4 Hz), 4.39 (1H, br s), 4.13 (1H, br s), 3.05 (1H, dd, J=7.9, 7.3 Hz), 2.34-2.17 (4H, m), 1.80-1.66 (2H, m).
$^{13}C$ NMR ($CDCl_3$) δ 152.4, 149.9, 112.1, 111.8, 63.2, 58.4, 45.5, 36.9, 28.4, 25.3.

**EXAMPLE 45**

2-(3,4,5-TRIMETHOXY-PHENYL)-7-AZA-BICYCLO[2.2.1]HEPTANE, HCL SALT

MP 255.5-270.0°C.
IR: (KBr) 2995.2, 2960.2, 2944.1, 2929.4, 2844.5, 2811.5, 2711.7, 2671.3, 2524.3, 2499.2, 1593.2, 1511.3, 1465.8, 1430.1, 1369.3, 1338.7, 1326.9, 1278.4, 1250.6, 1238.4, 1184.9, 1156.9, 1148.2, 1131.3, 1011.9, 1000.2, 945.9, 828.7, 733.0, 529.4, 510.5 $cm^{-1}$.
MS: CI ($M+H^+$) 264.2.
$^1H$ NMR ($CD_3OD$) δ 6.58 (2H, s), 4.42 (1H, d, J=3.3 Hz), 4.27 (1H, d, J=3.9 Hz), 3.85 (6H, d, J=1.0 Hz), 3.71 (3H, d, J=1.2 Hz), 3.32 (1H, dd, J=6.1, 9.4 Hz), 2.36 (1H, dd, J=9.5, 13.5 Hz), 2.10-1.84 (5H, m).
$^{13}C$ NMR δ 154.8, 138.8, 138.0, 105.4, 64.8, 61.1, 60.4, 56.9, 46.4, 37.8, 28.8, 26.8.

**EXAMPLE 46**

2-(5-NITRO-FURAN-2-YL)-7-AZA-BICYCLO[2.2.1]HEPTANE, HCL SALT

IR: (KBr) 3077.6, 3053.6, 2997.2, 2957.7, 2915.3, 2883.4, 2854.7, 2823.4, 2692.2, 2650.0, 2523.9, 1605.6. 1586.8, 1528.2, 1517.1, 1494.2, 1467.5, 1454.3, 1384.9, 1357.1, 1326.0, 1248.3, 1222.3, 1157.0, 1034.7, 809.8, 741.8 $cm^{-1}$.
MS: CI ($M+H^+$) m/z=209.1.
HRMS (m/z) 209.0912, calculated for $C_{10}H_{13}N_2O_3$, 209.0926.
$^1H$ NMR ($CD_3OD$) δ 7.40 (1H, d, J=3.5 Hz), 6.65 (1H, d, J=3.5 Hz), 4.51 (1H, d, J=3.5 Hz), 4.33 (1H, s), 3.52 (1H, dd, J=9.2, 5.5 Hz), 2.34 (1H, dd, J=13.5, 9.6 Hz), 2.22-2.19 (1H, m), 2.05-1.82 (4H, m).

**EXAMPLE 47**

5-(7-AZA-BICYCLO[2.2.1]HEPT-2-YL)-3-METHYL-BENZO[D]ISOXAZOLE, HCL SALT

MP: decomposes 267°C.
IR: (KBr), 2994.5, 2963.7, 2856.0, 2839.6, 2783.0, 2703.1, 2668.0, 2637.2, 2602.2, 2577.0, 2526.8, 2487.6, 1604.0, 1533.9, 1474.3, 1461.7, 1450.6, 1392.5, 1366.9, 1336.0, 1320.3, 1308.3, 1277.9, 1240.3, 1217.8, 1172.8, 1158.2, 911.7, 903.9, 893.3, 862.1, 845.4, 823.0, 797.0, 580.7, 560.0, 529.2, 512.2, 424.4 $cm^{-1}$.
MS: CI ($M+H^+$) m/z=229.2.
HRMS (m/z) 229.1356, calculated for $C_{14}H_{17}N_2O$, 229.1341.
$^1H$ NMR ($CD_3OD$) δ 7.76 (1H, d, J=0.8 Hz), 7.59-7.53 (2H, m), 4.53 (1H, d, J=3.3 Hz), 4.33 (1H, d, J=3.7 Hz), 3.54 (1H, dd, J=9.3, 6.0 Hz), 2.58 (3H, s), 2.46 (1H, dd, J=13.4, 9.7 Hz), 2.18-1.87 (5H, m).
$^{13}C$ NMR ($CD_3OD$) δ 163.2,156.7, 138.4, 131.4,123.7, 120.0, 111.0, 64.8, 60.5, 45.9, 38.1, 28.8, 26.9.

**EXAMPLE 48**

6-(7-AZA-BICYCLO[2.2.1]HEPT-2-YL)-3-METHYL-BENZO[D]ISOXAZOLE, HCL SALT

MP: decomposes 278°C.
IR: (KBr) 2990.9, 2954.3. 2925.9, 2879.8, 2859.4, 2825.1, 2714.4, 2690.8, 2652.3, 2548.8, 1619.0, 1611.7, 1602.1. 1464.0, 1450.7, 1435.5, 1415.7, 1393.9, 1372.4, 1365.6, 1353.5, 1327.6, 1309.8, 1266.4. 1165.0. 980.1, 939.1,

886.6, 855.4, 818.5, 798.3, 765.2, 675.2, 636.9, 438.4 cm-1.
MS: CI (M+H+) 229.2.
HRMS (m/z) 229.1346, calculated for $C_{14}H_{16}N_2O$, 229.1341.
$^1H$ NMR ($CD_3OD$) δ 7.76 (1H, d, J=8.3 Hz), 7.60 (1H, d, J=0.6 Hz), 7.31 (1H, dd, J=8.3, 1.2 Hz), 4.58 (1H, d, J=2.9 Hz), 4.31 (1H, d, J=4.2 Hz), 3.58 (1H, dd, J=9.5, 6.0 Hz), 2.54 (3H, s), 2.48 (1H, dd, J=13.4, 9.6 hz), 2.14-1.87 (5H, m).
$^{13}C$ NMR ($CD_3OD$) δ 164.7, 156.5, 145.9, 124.5, 123.2, 122.2, 108.3, 64.4, 60.5, 46.3, 38.1, 28.8, 26.9.

**EXAMPLE 49**

6-(7-AZA-BICYCLO[2.2.1]HEPT-2-YL)-1,4-DIHYDRO-QUINOXALINE-2,3-DIONE, HCL SALT

IR: (KBr) 3092.8, 3030.9, 2992.6, 2962.4, 2928.9, 2835.4, 2761.2, 2693.9, 2653.7, 1685.6, 1626.3, 1600.4, 1530.5, 1456.6, 1395.8, 1356.9, 1340.2, 1316.7, 1264.6, 894.3, 868.7, 851.6, 823.3, 769.8, 750.0, 742.8, 723.7, 686.5, 677.1, 647.6, 609.4, 583.9, 531.1, 470.3 $cm^{-1}$.
MS: CI (M+H+) m/z=258.2.
HRMS (m/z) 258.1250, calculated for $C_{14}H_{16}N_3O_2$, 258.1242.
$^1H$ NMR ($D_2O$) δ 6.75 (1H, d, J=8.5 Hz), 6.63 (1H, d, J=8.5 Hz), 6.50 (1H, s), 4.30 (1H, d, J=3.5 hz), 4.19 (1H, s), 3.03 (1H, ap. t.), 2.15 (1H, dd, J=13.2, 9.9 Hz), 1.93-1.67 (5H, m).
$^{13}C$ NMR ($D_2O$) δ 158.5, 158.1, 140.9, 126.8, 126.3, 125.5, 119.1, 116.4, 65.9, 62.2, 46.5, 37.9, 30.0, 28.6.

**EXAMPLE 50**

6-(7-AZA-BICYCLO[2.2.1]HEPT-2-YL)-QUINOXALINE, HCL SALT

MP: decomposes 240°C.
IR: (KBr) 3033.9, 2989.6, 2958.0, 2920.1, 2888.1, 2847.3, 2822.4, 2715.1, 2686.6, 2648.7, 2626.6, 2546.7, 2518.7, 1621.2, 1609.8, 1497.0, 1462.3, 1450.1, 1368.8, 1350.0, 1335.3, 1326.2, 1304.2, 1181.9, 1133.1, 1031.5, 980.6, 952.6, 901.5, 889.7, 870.9, 827.2, 524.2, 408.4 $cm^{-1}$.
MS: CI (M+H+) m/z=226.3.
$^1H$ NMR ($CD_3OD$) δ 8.89 (2H, d, J=11.1, 1.9 Hz), 8.11 (1H, d, J=8.8 Hz), 8.05 (1H, s), 7.82 (1H, dd, J=8.8, 2.1 Hz), 4.71 (1H, d, J=4.1 Hz), 4.37 (1H, t, J=4.4 Hz), 3.68 (1H, dd, J=9.5, 6.1 Hz), 2.55 (1H, dd, J=13.4, 9.7 Hz), 2.26-1.90 (5H, m).

**EXAMPLE 51**

1-[4-(7-AZA-BICYCLO[2.2.1]HEPT-2-YL)-2-FLUORO-PHENYL]-ETHANONE, HCL SALT

MP 180-183°C.
IR: (KBr) 3023.1, 2996.2, 2959.6, 2841.7, 2814.2, 2698.2, 2646.9, 2626.8, 2572.0, 2531.8, 2512.8, 1679.0, 1621.2, 1611.7, 1605.3, 1569.7, 1499.4, 1453.4, 1429.4, 1422.2, 1370.4, 1346.9, 1304.2, 1292.0, 1283.9, 1260.2, 1243.8, 1223.2, 1170.4, 1163.0, 1150.5, 1142.3, 1055.5, 965.0, 894.0, 878.5, 839.3, 775.3, 542.3, 523.9 cm-1.
MS: CI (M+H+) m/z=234.2.
$^1H$ NMR ($CD_3OD$) δ 7.86-7.82 (1H, m), 7.25-7.22 (2H, m), 4.54 (1H, d, J=3.6 Hz), 4.31 (1H, t, J=4.2 Hz), 3.46 (1H, dd, J=9.4, 6.0 Hz), 2.58 (3H, dd, J=4.5, 0.8 Hz), 2.44 (1H, dd, J=13.5, 9.6 Hz), 2.11-1.85 (5H, m).
$^{13}C$ NMR ($CD_3OD$) δ 163.8, 161.0, 149.53, 149.46, 130.70, 130.67, 122.81, 122.78, 114.99, 114.73, 62.5, 59.0, 44.4, 36.2, 29.8, 27.3, 25.4.

**PREPARATION 1**

7-CARBOETHOXY-2-CARBOXY-7-AZABICYCLO[2.2.1]-HEPTANE

A 1L round bottomed flask (RBF) was charged with 7-carboethoxy-2carboethoxy-7-azabicyclo[2.2.1]-heptane (28 g, 0.123 mol) and 250 mL THF.LiOH (8.8 g, 0.37 mol) was added in 86 mL $H_2O$ and the walls of the flask were rinsed with methanol (MeOH) (86 mL). The reaction was stirred at room temperature for 4 hours. The reaction mixture was partitioned between 1L ethyl acetate (EtOAc) and 200 mL $H_2O$. The organics were separated and extracted with 1N sodium hydroxide (NaOH) (5 x 200mL). The combined aqueous phases were reacidified with 6N HCl (ca. 62 mL), extracted with EtOAc (5 x 200mL), dried over $Na_2SO_4$, filtered through cotton, and concentrated *in vacuo* to an oil. The oil was dried under vacuum to afford the title product which was used without purification for the next step (24 g, 0.34 mol, 92%).

MS: Cl (m/z) 214 ($M+H^+$), 200 (60%), 186 (66%), 168 (87%).
$^1H$ NMR ($CD_3OD$) δ 4.45 (1H, m), 4.30 (1H, m) 4.15 (2H, q, J=7 Hz), 2.55 (1H, m), 1.75 (2H, m), 1.55 (1H, m), 1.44 (1H, br, s), 1.20 (3H, dd).

**PREPARATION 2**

7-CARBOETHOXY-7-AZABICYCLO[2.2.1]-HEPTENE

A 1L RBF was charged with 7-carboethoxy-2-carboxy-7-azabicyclo[2.2.1]-heptane (14.7 g, 68.9 mol) in 750 mL benzene. After purging with $N_2$, solid copper acetate (2.5 g, 13.8mol) was added (a blue hue emerged) followed by lead tetraacetate (39.7 g, 89.6mol). The reaction was stirred wrapped in aluminum foil overnight under $N_2$ overnight and then brought to reflux for 2 hours. The reaction mixture was filtered through paper paper, and the solid brown residue was rinsed with 1.1 hexane/ether (4 × 100mL). The blue filtrate was again filtered and the concentrated residue was then passed through a plug (with 1:1 hexane/ether) to afford 4.6 g pure title compound and 4.3 slightly impure title compound (total 8.9 g, 53.2 mol, 77% yield).

MS: Cl (m/z) 153 ($M+H^+$).
$^1H$ NMR ($CDCl_3$) δ 6.21 (2H, br,s), 4.71 (2H, br, s,) 4.05 (2H, q, J=7 Hz), 1.84 (2H, d, J=11 Hz), 1.19 (3H, dd, J=7.2,1Hz), 1.10 (2H, d, J=1 Hz).

**PREPARATION 3**

7-CARBO-*tert*-BUTOXY-7-AZABICYCLO[2.2.1]-HEPTENE

A 2-necked RBF equiped with a water-cooled condenser was flame-dried and charged with $N_2$ and a solution of 7-carboethoxy-7-azabicyclo[2.2.1]-heptene (1.0g, 6.01 mmol) in 10 mL $CHCl_3$. Triethylamine (TEA, 3.1 equiv., 2.59 ml) was added, followed by trimethylsilyl iodide (TMSI) (3.61g, 18 mmol, 3.0 equivalent (equiv.)), which was added dropwise, and the reaction was refluxed for 2 hours as the reaction color turned dark red. After cooling to room temperature, trifluoroacetic acid (TFA, 2.19 g, 1.48 mL, 19.2 mmol, 3.2 equiv.) was added and the reaction mixture was stirred at room temperature for 2 hours. After another addition of TEA (3.5 equiv.), t-butyl pyrocarbonate (2.61 g, 12.02 mmol) was added in 3.5 mL methylene chloride ($CHCl_3$), and the reaction was stirred overnight at room temperature. The reaction was worked up by partioning of the crude between 70 mL EtOAc and 30 mL water. The organics were separated of and washed with water (1 x 30 mL), dried ($Na_2SO_4$), filtered (paper), and concentrated *in vacuo* to afford a yellow solid. Flash chromatography (30g silicon dioxide ($SiO_2$), 90:10 hexane: ethyl acetate (EtOAc)) afforded the title product (0.850 g, 72%).

MS: Cl (m/z) 181 ($M+H^+$).
$^1H$ NMR ($CDCl_3$) δ 6.20 (2H, br,s), 4.64 (2H, br, s,) 1.83 (2H, br, s), 1.83 (2H, d, J=8.7 Hz), 1.45 (9H, s), 1.08 (2H, d, J=1 Hz).

## Claims

1. A compound of the formula

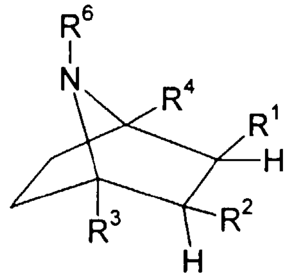

I

wherein

$R^1$, $R^2$, $R^3$ and $R^4$ are selected, independently from hydrogen, $-CO_2R^5$, aryl and heteroaryl, wherein said aryl is selected from phenyl and naphthyl and said heteroaryl is selected from pyrazinyl, benzofuranyl, quinolyl, isoquinolyl, benzothienyl, isobenzofuryl, pyrazolyl, indolyl, isoindolyl, benzimidazolyl, purinyl, carbazolyl, 1,2,5-thiadiazolyl, quinazolinyl, pyridazinyl, pyrazinyl, cinnolinyl, phthalazinyl, quinoxalinyl, xanthinyl, hypoxanthinyl, pteridinyl, 5-azacytidinyl, 5-azauracilyl, triazolopyridinyl, imidazolopyridinyl, pyrrolopyrimidinyl, and pyrazolopyrimidinyl oxazolyl, isoxazoyl, thiazolyl, isothiazolyl, furanyl, pyrazolyl, pyrrolyl, tetrazolyl, triazolyl, thienyl, imidazolyl, pyridinyl, and pyrimidinyl, and wherein said phenyl and said heteroaryl may optionally be substituted with from one to three substitutuents, and are preferably substituted with one or two substutituents, independently selected form $(C_1-C_6)$alkyl optionally substituted with from one to seven (preferably with from zero to four) fluorine atoms, halo (i.e., chloro, fluoro, bromo or iodo), phenyl, benzyl, hydroxy, acetyl, amino, cyano, nitro, $(C_1-C_6)$alkoxy optionally substituted with from one to seven (preferably with from zero to four) fluorine atoms, $(C_1-C_6)$alkylamino and $[(C_1-C_6)alkyl]_2$amino;
$R^5$ is $(C_1-C_6)$ alkyl, aryl, heteroaryl, $(C_1-C_4)$alkylene-aryl and $(C_1$-C4)alkylene-heteroaryl, wherein said aryl and heteroaryl are defined as above, and wherein said $(C_1-C_6)$alkyl may optionally be substituted with from one to three substituents independently selected from halo, $(C_1-C_6)$alkyl, $(C_1-C_6$ )alkoxy, $(C_1-C_4)$alkoxy-$(C_1-C_4)$alkyl, amino, $(C_1-C_6)$alkylamino, and $[(C_1-C_6)alkyl]_2$amino; and
$R^6$ is hydrogen or $(C_1-C_6)$alkyl;

with the proviso that: (a) at least one of $R^1$, $R^2$, $R^3$, and $R^4$ must be aryl or heteraryl; (b) when neither $R^1$ nor $R^2$ is hydrogen, $R^1$ and $R^2$ are in the "exo" configuration; (c) $R^1$ and $R^2$ can not both be $-CO_2R^5$; (d) if either $R^3$ or $R^4$ is $CO_2R^5$ and $R^5$ is an alkyl or alkoxyalkyl group, then one of $R^1$ and $R^2$ must be aryl or heteroaryl; and (e) if either $R^1$ or $R^2$ is $CO_2R^5$ and $R^5$ is an alkyl or alkoxyalkyl group, then one of $R^3$ and $R^4$ must be aryl or heteroaryl;
or a pharmaceutically acceptable salt thereof.

**2.** A compound according to claim 1, wherein $R^3$ and $R^4$ are hydrogen, and one of $R^1$ and $R^2$ is optionally substituted phenyl and the other is hydrogen.

**3.** A compound according to claim 1, wherein $R^3$ and $R^4$ are hydrogen, and one of $R^1$ and $R^2$ is phenyl substituted with fluoro or nitro and the other is hydrogen.

**4.** A compound according to claim 1, wherein $R^3$ and $R^4$ are hydrogen and one of $R^1$ and $R^2$ is hydrogen and the other is: (a) 3-fluorophenyl; (b) 4-nitrophenyl; or 3-fluoro-4-nitrophenyl.

**5.** A pharmaceutical composition for use in reducing nicotine addiction or aiding in the cessation or lessening of tobacco use in a mammal, comprising an amount of a compound according to claim 1 that is effective in reducing nicotine addiction or aiding in the cessation or lessening of tobacco use and a pharmaceutically acceptable carrier.

**6.** A method for reducing nicotine addiction or aiding in the cessation or lessening of tobacco use in a mammal, comprising administering to said mammal an amount of a compound according to claim 1 that is effective in reducing nicotine addiction or aiding in the cessation or lessening of tobacco use.

**7.** A pharmaceutical composition for treating a disorder or condition selected from inflammatory bowel disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amylotropic lateral sclerosis (ALS), cognitive dysfunction, hypertension, bulimia, anorexia, obesity, cardiac arrythmias, gastric acid hypersecretion,

ulcers, pheochromocytoma, progressive supramuscular palsy, chemical dependencies and addictions, headache, stroke, TBI, psychosis, Huntington's Chorea, tardive dyskinesia, hyperkinesia, dyslexia, schizophrenia, multi-infarct dementia, age related cognitive decline, epilepsy, including petit mal absence epilepsy, senile dementia of the Alzheimer's type (AD), Parkinson's disease (PD), attention deficit hyperactivity disorder (ADHD) and Tourette's Syndrome in a mammal, comprising an amount of a compound according to claim 1 that is effective in treating such disorder or condition and a pharmaceutically acceptable carrier.

**8.** A method for treating a disorder or condition selected from inflammatory bowel disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amylotropic lateral sclerosis (ALS), cognitive dysfunction, hypertension, bulimia, anorexia, obesity, cardiac arrythmias, gastric acid hypersecretion, ulcers, pheochromocytoma, progressive supramuscular palsy, chemical dependencies and addictions, headache, stroke, TBI, psychosis, Huntington's Chorea, tardive dyskinesia, hyperkinesia, dyslexia, schizophrenia, multi-infarct dementia, age related cognitive decline, epilepsy, including petit mal absence epilepsy, senile dementia of the Alzheimer's type (AD), Parkinson's disease (PD), attention deficit hyperactivity disorder (ADHD) and Tourette's Syndrome in a mammal, comprising administering to a mammal in need of such treatment an amount of a compound according to claim 1 that is effective in treating such disorder or condition.

**9.** A process for preparing a compound of the formula

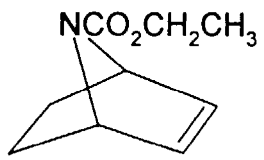

XVI

comprising reacting a compound of the formula

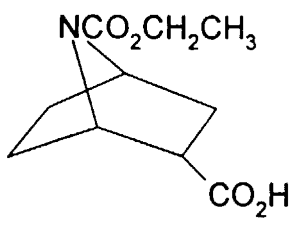

XVII

with lead tetraacetate and copper acetate.

**10.** A process according to claim 9 which is conducted at the reflux temperature using benzene, toluene or xylenes as the solvent.

**11.** A process according to claim 10 which is conducted using benzene as the solvent.